# EUROPEAN PATENT APPLICATION

(11) **EP 3 235 813 A1**
(43) Date of publication of application: **25.10.2017**
(21) Application number: 16382175.4
(22) Date of filing: 19.04.2016
(51) Int. Cl.: C07D 401/14, A61K 31/403, A61K 31/4439, A61K 31/454, C07D 401/06, C07D 409/06, C07D 409/14, C07D 209/70, C07D 209/72, A61P 3/00, A61P 9/00, A61P 25/00, A61P 29/00

(54) **AZA-TETRA-CYCLO DERIVATIVES**

(71) Applicant: CIDQO 2012, S.L., 08490 Tordera - Barcelona (ES); Universitat de Barcelona, 08028 Barcelona (ES)
(72) Inventor: VÁZQUEZ CRUZ, Santiago, E-08940 Cornellá - Barcelona (ES); LEIVA MARTÍNEZ, Rosana, E-08028 Barcelona (ES); VALVERDE MURILLO, Elena, E-43711 Banyeres del Penedès - Tarragona (ES)
(74) Representative: ABG Patentes, S.L.

(57) **Abstract**

The present invention relates to compounds of formula (I) 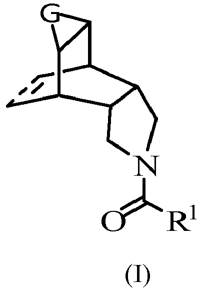 which are capable of inhibiting the 11-beta-hydroxysteroid dehydrogenase type 1 (11-beta-HSD1), to processes for preparing these compounds, to pharmaceutical compositions comprising an effective amount of these compounds and to the use of the compounds in medicine in particular for the treatment of pathological conditions or diseases that can improve by inhibition of 11-beta-HSD1.

## Description

### FIELD OF THE INVENTION

The invention relates to new aza-(tri or tetra)-cyclo derivatives capable of inhibiting the 11-beta-hydroxysteroid dehydrogenase type 1 (11-beta-HSD1).

Other objectives of the present invention are to provide a procedure for preparing these compounds; pharmaceutical compositions comprising an effective amount of these compounds; the use of the compounds for manufacturing a medicament for the treatment of pathological conditions or diseases that can improve by inhibition of 11-beta-HSD1, such as obesity, insulin resistance, metabolic syndrome, cognitive disfunction associated to Alzheimer's disease, HIV-associated dementia, Creutzfeld-Jakob disease, Parkinson's disease, Huntington's disease, vascular inflammation, atherosclerosis, diabetes type 2, cardiovascular disease, glaucoma, osteoporosis, myocardial infarction and for alcohol abuse treatment.

### BACKGROUND OF THE INVENTION

Glucocorticoids (GCs) are well-known ubiquitous hormones playing a key role in modulating immune and inflammatory responses, regulating energy metabolism and cardiovascular homeostasis and are a key element of stress response. GCs act through the intracellular glucocorticoid receptors and, in some tissues, through mineralocorticoid receptors, both being nuclear transcription factors. The action of GCs on the target tissues depends not only on circulating steroid concentrations and the cellular expression of the receptors, but also on the intracellular enzymes which critically determine up to what point the glucocorticoids will have active access to the receptors. Opposing the action of insulin, GCs promote gluconeogenesis, inhibit β-cell insulin secretion and peripheral glucose uptake. Moreover, GCs contribute to lipolysis, with subsequent fatty acid mobilization, and proteolysis. Thus, overall, GCs prompt catabolic states (e.g., see Dallman, M. F. et al. "Feast and famine: critical role of glucocorticoids with insulin in daily energy flow" in Front. Neuroendocrinol. 1993, 14, 303-347, and Sapolsky, R. M. et al. "How do glucocorticoids influence stress responses? Integrating permissive, suppressive, stimulatory, and preparative actions" in Endocr. Rev. 2000, 21, 55-89).

The active human GC cortisol, is synthesized by the adrenal cortex with a strong circadian rhythm. Its secretion is controlled by the hypothalamic-pituitary-adrenal (HPA) axis and induced in stressful conditions. Serum cortisol readily passes cell membranes and exerts its intracellular functions by binding to GC receptors (GRs), which are ligand-activated nuclear receptors. GRs regulate, directly or indirectly, the expression of a plethora of genes involved in various physiological processes (e.g., see Rosen, E. D. "Adipocyte differentiation from the inside out" in Nat. Rev. Mol. Cell. Bio. 2006, 7, 697-706). Furthermore, GCs bind to mineralocorticoid receptors, which regulate blood pressure and sodium balance in the kidney (see Arriza, J. L. et al. "Cloning of human mineralocorticoid receptor complementary DNA: structural and functional kinship with the glucocorticoid receptor" in Science 1987, 237, 268-275).

Along with the HPA axis, GC's homeostasis is controlled by the 11β-hydroxysteroid dehydrogenase enzyme. Two isoforms of this enzyme, 11β-HSD type 1 (11β-HSD1) and type 2 (11βP-HSD2), catalyse the interconversion between active 11-hydroxy forms (cortisol in humans, and corticosterone in rodents) and inert 11-ketosteroids (cortisone in humans, and 11-dehydrocorticosterone in rodents). The 11β-HSD1 enzyme, mainly expressed in liver, adipose tissue, bone, pancreas, vasculature and brain, reconverts inactive glucocorticoids into active ones, thus playing an important role in modulating cellular agonist concentration and, therefore, in activating corticosteroid receptors in the target tissues.

### METABOLIC DISEASE SYNDROME AND DIABETES

Type 2 diabetes mellitus (T2DM) is characterized by increased cellular insulin resistance with an initial phase of enhanced insulin secretion followed by pancreatic β-cell failure. Regarding obesity, a person with a body mass index of 30 or more is considered obese, entailing health risks. T2DM and obesity are considered as first-world epidemics and are nowadays great healthcare challenges in the developed countries. According to the World Health Organization (WHO), in 2014, 9% of the worldwide adult population had diabetes (see WHO web page. Diabetes Fact Sheet, http://www.who.int/mediacentre/factsheets/fs312/en/ accessed on 21st March 2016). That same year, the percentage of total health expenditure in T2DM reached US $ 612 billion. The expected increase on the diabetic population by 2035 is about 205 million, which will elevate the total health cost still higher (see, European Comission web page. Major and chronic diseases: Diabetes. http://ec.europa.eu/health/major_chronic_diseases/diseases/diabetes/index_en.htm#frag ment7 accessed on 21st March 2016). Concerning obesity, in 2014 more than 1.9 billion adults were overweight, and of these, 600 million were obese (WHO web page. Obesity and overweight Fact Sheet. http://www.who.int/mediacentre/factsheets/fs311/en/ accessed on 21st March 2016).

From another standpoint, the metabolic syndrome is a cluster of metabolic abnormalities including resistance to insulin, obesity, hyperglycemia, and hypertension that represents a major risk factor for type-2 diabetes and cardiovascular disease. It is diagnosed in a given patient if visceral obesity and two or more risk factors are present, including elevated fasting plasma glucose, elevated triglycerides, elevated blood pressure and reduced high-density lipoprotein (see Huang, P. L. "A comprehensive definition for metabolic syndrome" in Dis. Model Mech. 2009, 2, 231-237"). Among the mechanisms involved in its pathogenesis, glucocorticoid synthesis and metabolism have been proposed to play a key role (e.g., see Wamil, M. et al. "Inhibition of 11β-hydroxysteroid dehydrogenase type 1 as a promising therapeutic target" in Drug Discover. Today 2007, 12, 504-520).

Currently, the predominant therapeutic strategy for preventing or managing T2DM and metabolic syndrome is the individual treatment of the set of risk factors instead of treating the disorder as a whole. The increase in the prevalence of T2DM and metabolic syndrome over the last few decades in the developed countries has propelled a growing need for the development of novel therapies to treat metabolic syndrome and its associated disorders.

Evidence has accumulated that enzyme activity of 11β-hydroxysteoid dehydrogenase type 1 (11β-HSD1), which regenerates active glucocorticoids from inactive forms and plays a central role in regulating intracellular glucocorticoid concentration, is commonly elevated in fat depots from obese individuals. Notably, increased tissue 11β-HSD1 expression and activity have been demonstrated in metabolic tissues including liver and adipose in human obesity and metabolic syndrome.

The impact of tissue-specific GC activation by 11β-HSD1 in metabolic homeostasis has been examined using several transgenic mouse models. Experiments in mice with adipose tissue-targeted 11β-HSD1 overexpression developed a phenotype similar to the one of Cushing's syndrome, including insulin resistance, visceral obesity, dyslipidaemia and hypertension, without an increase in the GC plasmatic levels (see, Masuzaki, H. et al. "A transgenic model of visceral obesity and the metabolic syndrome" in Science 2001, 294, 2166-2170, and Masuzaki, H. et al. "Transgenic amplification of glucocorticoid action in adipose tissue causes high blood pressure in mice" in J. Clin. Invest. 2003, 112, 83-90). On the other hand, overexpression of 11β-HSD1 in liver tissue led to the appearance of metabolic syndrome but without impaired glucose tolerance and obesity (see Paterson, J. M. et al. "Metabolic syndrome without obesity: hepatic overexpression of 11beta-hydroxysteroid dehydrogenase type 1 in transgenic mice" in Proc. Natl. Acad. Sci USA 2004, 101, 7088-7093). Conversely, 11ß-HSD1 knockout (11β-HSD1 -/-) mice resist visceral fat accumulation and insulin resistance even on a high-fat diet and have a 'cardioprotective' phenotype (see Kotelevtsev, Y. et al. "11beta-hydroxysteroid dehydrogenase type 1 knockout mice show attenuated glucocorticoid-inducible responses and resist hyperglycemia on obesity or stress" in Proc. Natl. Acad. Sci. 1997, 94, 14924-14929, Morton, N. M. et al. "Novel adipose tissue-mediated resistance to diet-induced visceral obesity in 11beta-hydroxysteroid dehydrogenase type-1 deficient mice" in Diabetes 2004, 53, 931-938, and Morton, N. M. et al. "Improved lipid and lipoprotein profile, hepatic insulin sensitivity, and glucose tolerance in 11beta-hydroxysteroid dehydrogenase type 1 null mice" in J. Biol. Chem. 2001, 276, 41293-41300), whose effects are also seen with 11ß-HSD1 inhibitors in rodents (see, Wamil, M. and Seckl, J. R. "Inhibition of 11β-hydroxysteroid dehydrogenase type 1 as a promising therapeutic target" in Drug Discover. Today 2007, 12, 504-520).

Several studies have appeared over the recent years proving the relationship between tissue-specific alterations in 11β-HSD1 expression in human T2DM, obesity and metabolic syndrome (see, Morgan, S. A. et al. "11beta-hydroxysteroid dehydrogenase type 1 inhibitors for the treatment of type 2 diabetes" in Expert Opin. Investig. Drugs 2010, 19, 1067-1076). For instance, the expression of 11β-HSD1 in human adipose tissue is positively correlated with the degree of obesity (e.g., see Valsamakis, G. et al. "11beta-hydroxysteroid dehydrogenase type 1 activity in lean and obese males with type 2 diabetes mellitus" in J. Clin. Endocrinol. Metab. 2004, 89, 4755-4761 and Wang, M. "Tissue-specific glucocorticoid excess in the metabolic syndrome: 11β-HSD1 as a therapeutic target" in Drug Develop. Res. 2006, 67, 567-569).

Hence, modulation of 11β-HSD enzyme, and in particular inhibition of 11β-HSD1 in adipose tissue and liver, are of special interest as pharmacotherapeutic strategies in the medical treatment of the metabolic syndrome and associated disorders (see Masuzaki, H. et al. "Tissue-specific glucocorticoid reactivating enzyme, 11β-hydroxysteroid dehydrogenase type 1 (11β-HSD1) - a promising drug target for the treatment of metabolic syndrome" in Curr. Drug Targets Immune Endocr. Metabol. Discord. 2003, 3, 255-262, Morton, N. M. "Obesity and corticosteroids: 11beta-hydroxysteroid type 1 as a cause and therapeutic target in metabolic disease" in Mol. Cell. Endocrinol. 2010, 316, 154-164, Anderson, A. et al. "11β-HSD1 inhibitors for the treatment of type 2 diabetes and cardiovascular disease" in Drugs 2013, 73, 1385-1393, and Wang, M. "Inhibitors of 11β-Hydroxysteroid Dehydrogenase Type 1 in Antidiabetic Therapy" in Diabetes - Perspectives in Drug Therapy, Schwanstecher, M. (ed.), Handbook of Experimental Pharmacology 203, 2011, 127-146, Springer-Verlag).

Numerous inhibitors of 11β-HSD enzymes have been identified. Naturally occurring inhibitors include the liquorice derivative glycyrrhetinic acid, flavanone/hydroxyl-flavanone, bile acids, and progesterone metabolites; however, most of these compounds inhibit both 11β-HSD1 and 11β-HSD2, and as a result their therapeutic benefit is limited due to ensuing hypertension and hypokalemia (see Gathercole, L. L. "11β-Hydroxysteroid dehydrogenase 1: translational and therapeutic aspects" in Endocrine Rev. 2013, 34, 525-555). In humans, the first published reports of the metabolic consequences of 11β-HSD1 inhibition were derived from the nonselective compound, carbenoxolone. In healthy individuals, carbenoxolone improves whole body insulin sensitivity, and in patients with type 2 diabetes it decreases glucose production rates, principally through a decrease in glycogenolysis with no apparent effect on gluconeogenesis. In addition, it decreases total circulating cholesterol (see Andrews, R. C. et al. "Effects of the 11-hydroxysteroid dehydrogenase inhibitor carbenoxole on insulin sensitivity in men with type 2 diabetes" in J. Clin. Endocrinol. Metab. 2003, 88, 285-291).

Highly potent selective 11β-HSD1 inhibitors have been developed by several major pharmaceutical companies such as Abbott, Amgen, Astellas, Bristol-Myers-Squibb, Daiichi Sankyo, Merck, Novo Nordisk, AstraZeneca, Sterix, Shionogi, Biovitrum, Janssen, Pfizer, Takeda, Lilly, Taisho, Wyeth, Ono, Incyte, Roche and Novartis (see, Fotsch, C.; Askew, B. C.; Chen, J. J. "11β-Hydroxysteroid dehydrogenase-1 as a therapeutic target for metabolic diseases" in Expert Opin. Ther. Patents 2005, 15, 289-303, and Boyle, C. D.; Kowalski, T. J. "11β-hydroxysteroid dehydrogenase type 1 inhibitors: a review of recent patents" in Expert Opin. The. Patents 2009, 19, 801-825).

In clinical trials, 11β-HSD1 inhibitors have been well tolerated and have improved glycaemic control, lipid profile and blood pressure, and induced modest weight loss (see, Anderson, A.; Walker, B. R. "11β-HSD1 inhibitors for the Treatment of Type 2 Diabetes and Cardiovascular Disease", in Drugs 2013, 73, 1385-1393). Proof of concept for the 11β-HSD1 inhibitor class was demonstrated with INCB13739. In patients with T2DM failing metformin monotherapy, INCB13739 treatment achieved significant reductions in haemoglobin Alc and fasting plasma glucose, and when present improves hyperlipidaemia and hypertriglyceridaemia (see, Hollis, G.; Huber, R. "11β-Hydroxysteroid dehydrogenase type 1 inhibition in type 2 diabetes mellitus" in Diabetes, Obes. Metab. 2011, 13, 1-6). More recently, novel 11β-HSD1 inhibitors have been progressed into clinical trials (e. g., see Scott, J. S. "Medicinal chemistry of inhibitors of 11β-hydroxysteroid dehydrogenase type 1 (11β-HSD1)" in J. Med. Chem. 2014, 57, 4466-4486). For example, in a phase 1b trial, RO5093151 was effective and safe in reducing liver-fat content in overweight, insulin-resistant patients with liver steatosis (see Stefan, N. "Inhibition of 11β-HSD1 with RO5093151 for non-alcoholic fatty liver disease: a multicentre, randomised, double-blind, placeo-controlled trial" in Lancet Diabetes Endocrinol. 2014, 2, 406-416). Another inhibitor, RO5027838, in a phase 2 study in metformin-treated patients with T2DM over 28 days showed inhibitory effects on 11β-HSD1 activity. This molecule showed a trend for improved haemoglobin Alc levels and consistent reduction of body weight exceeding that observed with placebo (see Heisel, T. et al. "Safety, efficacy and weight effect of two 11β-HSD1 inhibitors in metformin-treated patients with type 2 diabetes" in Diabetes Obes. Meta. 2014, 16, 1070-1077). However, most of 11β-HSD1 inhibitor development programs have been discontinued, predominantly due to insufficient efficacy. Therefore novel 11β-HSD1 inhibitors are still required.

### VASCULAR INFLAMMATION AND ATHEROSCLEROSIS

Several lines of evidence points towards 11β-HSD1 inhibition as an attractive target in atherosclerosis and vascular inflammation (see, e.g. Hadoke, P. W. F. et al. "Modulation of 11β-hydroxysteroid dehydrogenase as a strategy to reduce vascular inflammation" in Curr. Atheroscler. Rep. 2013, 15:320). Inhibition of 11β-HSD1 with compound MK-544 slowed plaque progression in a murine model of atherosclerosis, the key clinical sequela of metabolic syndrome. Mice with a targeted deletion of apolipoprotein E exhibited 84% less accumulation of aortic total cholesterol, as well as lower serum cholesterol and triglycerides, when treated with an 11β-HSD1 inhibitor. These data provide the first evidence that pharmacologic inhibition of intracellular GC activation can effectively treat atherosclerosis, the key clinical consequence of metabolic syndrome, in addition to its salutary effect on multiple aspects of the metabolic syndrome itself (see, Hermanowski-Vosatka A. et al. "11β-HSD1 inhibition ameliorates metabolic syndrome and prevents progression of atherosclerosis in mice" in J. Exp. Med. 2005, 202, 517-527). More recently, a similar reduction in intra-aortic cholesterol has been shown with a distinct inhibitor (L-750) (Luo, M. J. et al. "11β-HSD1 inhibition reduces atherosclerosis in mice by altering proinflammatory gene expression in the vasculature" in Physiol. Genomics 2013, 45, 47-57). Additionally, it has been found that levels of 11β-HSD1 mRNA are increased in human atherosclerotic vessels compared either with nearby intact (atheroma-free) vasculature or with vessels from control patients without coronary artery disease (Atalar, F. et al. "11β-hydroxysteroid dehydrogenase type 1 gene expression is increased in ascending aorta tissue of metabolic syndrome patients with coronary artery disease" in Genet. Mol. Res. 2012, 11, 3122-3132; Ayari, H. et al. "Auto-amplification of cortisol actions in human carotid atheroma is linked to arterial remodelling and stroke" in Fundam. Clin. Pharmacol. 2014, 28, 53-64).

### COGNITIVE DYSFUNCTION AND RELATED DISORDERS SUCH AS VASCULAR DEMENTIA

In the CNS, 11β-HSD1 is highly expressed in regions important for cognition such as hypothalamus, hippocampus, frontal cortex, and cerebellum (see, e.g., Moisan et al., "11beta-hydroxysteroid dehydrogenase bioactivity and messenger RNA expression in rat forebrain: localization in hypothalamus, hippocampus, and cortex" in Endocrinology 1990, 127, 1450-1455). Growing evidence suggests excessive glucocorticoid activity may contribute to Alzheimer's disease (AD) and age-associated memory impairment. It is known that glucocorticoids locally generated by 11beta-HSD1 play a major role in age-related cognitive impairments (MacLullich, A. M. J. et al., "11β-hydroxysteroid dehydrogenase type 1, brain atrophy and cognitive decline" in Neurobiol. Aging 2012, 33, 207.e1-207.e8) and that 11beta-HSD1 deficiency prevents memory deficits with aging (see, e.g., Yau, J. L. W. et al. "Lack of tissue glucocorticoid reactivation in 11β-hydroxysteroid dehydrogenase type 1 knockout mice ameliorates age-related learning impairments" in Proc. Natl. Acad. Sci. U.S.A. 2001, 98, 4716-4721). Also, it has been found that aging associates with elevated hippocampal and neocortical 11beta-HSD1 and impaired spatial learning while deficiency of 11beta-HSD1 in knockout mice prevents the emergence of cognitive decline with age (see, e.g., Yau, J. L. W. et al. "Local amplification of glucocorticoids in the aging brain and impaired spatial memory" in Frontiers Aging Neurosci. 2012, 4, 24). Thus, mice with lifelong deficiency of 11beta-HSD1 resist age-dependent spatial memory impairments even though plasma corticosterone levels are elevated to the same level as wildtype mice (see, e.g., Yau, J. L. W. et al. "Lack of tissue glucocorticoid reactivation in 11β-hydroxysteroid dehydrogenase type 1 knockout mice ameliorates age-related learning impairments" in Proc. Natl. Acad. Sci. U.S.A. 2001, 98, 4716-4721). This occurs in various cognitive tasks of spatial memory, such as the Morris water maze and the Y-maze (see, e.g., Yau, J. L. W. et al. "Enhanced hippocampal long-term potentiation and spatial learning in aged 11beta-hydroxysteroid dehydrogenase type 1 knockout mice" in J. Neurosci. 2007, 27, 10487-10496). Conversely transgenic mice with modest forebrain-specific overexpression of 11beta-HSD1 show accelerated memory deficits with ageing (see, e.g., Holmes, M. C. et al., "11beta-Hydroxysteroid dehydrogenase type 1 expression is increased in the aged mouse hippocampus and parietal cortex and causes memory impairments" in J. Neurosci. 2010, 19, 6916-6920).
Furthermore, short-term pharmacological inhibition of 11beta-HSD1 in already aged mice reverses spatial memory impairments (see, e.g., Sooy, K. et al. "Partial deficiency or short-term inhibition of 11beta-hydroxysteroid dehydrogenase type 1 improves cognitive function in aging mice" in J. Neurosci. 2010, 30, 13867-13872; Yau, J. L. W. et al. "Intrahippocampal glucocorticoids generated by 11beta-HSD1 affect memory in aged mice" in Neurobiol. Aging 2015, 36, 334-343; Wheelan, N. et al. "Short-term inhibition of 11beta-hydroxysteroid dehydrogenase type 1 reversibly improves spatial memory but persistently impairs contextual fear memory in aged mice" in Neuropharmacol. 2015, 91, 71-76. Also, in a well-characterized murine AD model, the Tg2576 mouse, which bears a mutated human amyloid precursor protein (APP) gene, a short-term treatment (four weeks) with a known 11beta-HSD1 inhibitor improves cognition and a long-term (44 weeks) prevents cognitive decline (see, e.g., Sooy, K. et al. "Cognitive and disease-modifying effects of 11beta-hydroxysteroid dehydrogenase type 1 inhibition in male Tg2576 mice, a model of Alzheimer's disease" in Endocrinology 2015, 156, 4592-4603). Finally, Amgen has also disclosed that aged rats treated with another selective 11beta-HSD1 inhibitor, AMG221, exhibited superior improvement in the novel object recognition test, when compared to rats that had been administered anti-AD drug galantamine (WO2012051139A1). Finally, in humans, four weeks of the non-selective 11beta-hydroxysteroid dehydrogenase inhibitor carbenoxolone improved verbal fluency in healthy elderly men and type 2 diabetics who have a selective impairment in verbal memory (Sandeep, T. C. et al. "11beta-hydroxysteroid dehydrogenase inhibition improves cognitive function in healthy elderly men and type 2 diabetics" in Proc. Natl. Acad. Sci. USA 2004, 101, 6734-6739)*.*

Also, increased cortisol levels after an overnight dexamethasone-suppresion test have been reported in vascular dementia and depression, suggesting a beneficial role of 11b-HSD1 inhibitors in these diseases (see, e.g., Martocchia, A. et al. "Recent advances in the role of cortisol and metabolic syndrome in age-related degenerative diseases" in Aging Clin. Exp. Res. 2016, 28, 17-23).

Thus, 11 β-HSDI inhibitors are of potential therapeutic utility in the treatment of diseases which are characterised by cognitive impairment such as Alzheimer' disease and related neurodegenerative diseases.

### GLAUCOMA

Glucocorticoid target receptors and the enzymes regulating glucocorticoid activity at these receptors are present in mammalian ocular tissues, which regulate aqueous humor formation and outflow. Alteration in the number or affinity of receptors or in the activity of regulatory enzymes may alter the susceptibility of certain individuals to the effects of glucocorticoids on intraocular pressure. Glucocorticoids increase the risk of glaucoma by increasing intraocular pressure when they are exogenously administered and in certain conditions of increased production such as Cushing's syndrome (see, Stokes, J. et al. "Distribution of glucocorticoid and mineralocorticoid receptors and 11β-hydroxysteroid dehydrogenases in human and rat ocular tissues" in Invest. Invest. Ophthalmol. Vis. Sci. 2000, 41, 1629-1638). It has been found that the intraocular pressure can be decreased significantly in humans after few days of oral treatment with carbenoxolone, a known 11β-HSD1 inhibitor (see, Rauz, S. et al. "Expression and putative role of 11β-hydroxysteroid dehydrogenase isozymes within the human eye" in Invest. Ophthalmol. Vis. Science 2001, 42, 2037-2042). Also, carbenoxolone lowers intraocular pressure in patients with ocular hypertension mediated through inhibition of 11β-HSD1 in the ciliary epithelium (Rauz, S. et al. "Inhibition of 11β-hydroxysteroid dehydrogenase type 1 lowers intraocular pressure in patients with ocular hypertension" in Q. J. Med. 2003, 96, 481-490). Thus, selective and topical inhibitors of 11β-HSD1 could provide a treatment for patients with glaucoma.

### OSTEOPOROSIS

Osteoporosis is characterized by decreased bone mineral density which leads to bone fragility and increased fracture risk due to reduced bone strength. This is due to the imbalance of the bone remodeling process. Glucocorticoids are crucial for normal bone development but are detrimental in excess. Rapid onset of bone loss and fragility fractures are common complications of long term systemic glucocorticoids treatment and glucocorticoid induced osteoporosis affects up to 50 % of patients taking long term glucocorticoid therapy (e.g., see Raqisz, L. G. "Pathogenesis of osteoporosis: concepts, conflicts, and prospects" in J. Clin. Invest. 2005, 115, 3318-3325). Glucocorticoid action in bone depends upon the activity of 11β-HSD1 enzyme. Regulations of 11β-HSD1 activity may protect the bone against bone loss due to excess glucocorticoids. Treatment with glycyrrhizinc acid, a potent 11β-HSD inhibitor, led to significant reduction in bone resorption marker pyridinoline and improved the bone structure and biomechanical strength of rats receiving long-term glucocorticoids therapy (Ramli, E. S. M. et al. "Glycyrrhizic acid (GCA) as 11β-hydroxysteroid dehydrogenase inhibitor exerts protective effect against glucocorticoid-induced osteoporosis" in J. Bone Miner. Metab. 2013, 31, 262-273). In another study, BVT-2733, an 11β-HSD1 selective inhibitor, protected osteoblasts against endogenous glucocorticoid induced dysfunction (see Wu, L. et al. "11β-hydroxysteroid dehydrogenase type 1 selective inhibitor BVT.2733 protects osteoblasts against endogenous glucocorticoid induced dysfunction" in Endocrine J. 2013, 60, 1047-1058). Also, KR-67500, a potent 11β-HSD1 inhibitor, enhances the osteolastogenesis and inhibits the osteoclastogenesis (see Park, J. S. et al. "A novel 11β-HSD1 inhibitor improves diabesity and osteoblast differentiation" in J. Mol. Endocrinol. 2014, 52, 191-202). Therefore, 11β-HSD1 inhibitors have the potential to be used as new therapeutic agents against osteoporosis.

### ANGIOGENESIS AND MYOCARDIAL INFARCTION:

Angiogenesis restores blood flow to healing tissues, a process that is inhibited by high doses of glucocorticoids. Endogenous glucocorticoids, including those generated locally by 11β-HSD1, exert tonic inhibition of angiogenesis. 11β-HSD1 knockout mice showed enhanced angiogenesis *in vitro* and *in vivo* within sponges, wounds, and infarcted myocardium (e.g., see McSweeney, S. et al. "Improved heart function enhanced inflammatory cell recruitment and angiogenesis in 11β-HSD1-deficient mice post-MI" in Cardiovasc. Res. 2010, 88, 159-167). Thus, inhibition of 11β-HSD1 may offer a therapeutic approach to improve healing of ischemic or injured tissue, such as in ischemic heart diseases and impaired wound healing (see, Small, G.R. et al. "Preventing local regeneration of glucocorticoids by 11ß-hydroxysteroid dehydrogenase type 1 enhances angiogenesis" in Proc. Natl. Acad. Sci. U. S. A. 2005, 102, 12165-12170). Recently it has been found that UE2316, a selective 11β-HSD1 inhibitor, increases angiogenesis and improves cardiac function after myocardial infarction in mice (see, McGregor, K. et al. "Immediate pharmacological inhibition of local glucocorticoid generation increases angiogenesis and improves cardiac function after myocardial infarction" in Heart 2014, 100 (supp 3), A118).

### TREATMENT OF ALCOHOL ABUSE:

Few phamacotherapies for alcohol abuse, the most prevalent abused substance in the United Stantes and Europe, are currently available, and these have shown only limited efficacy and compliance. Thus, the development of more effective medications for alcohol abuse is a significant unmet medical need (e.g., see Litten, R. Z. et al. "Medications development to treat alcohol dependence: a vision for the next decade" in Addict Biol. 2012, 17, 513-527). Alcohol intake activates glucocorticoids. As glucocorticoid effects are modulated in part by the activity of 11β-HSD, its inhibition may be benefitial for the treatment of alcohol abuse. In fact, the 11-HSD inhibitor carbenoxolone reduces both baseline and excessive drinking in rodents (see Sanna, P. P. et al. "11b-hydroxysteroid dehydrogenase inhibition as a new potential therapeutic target for alcohol abuse" in Transl. Psychiatry 2016, 6, e760).

The patent literature describe numerous examples of 11-beta-hydroxysteroid dehydrogenase type 1 (11-beta-HSD1) inhibitors. The following are a few examples: WO 2015/077286, WO 2013/025664, WO 2011/161128, WO 2011/068927, WO 2012/061708, WO 2011/049520, WO 2011/002910, WO 2011/011123, WO 2010/139673, WO 2010/141424, WO 2010/089303, WO 2010/091067, WO 2010/022850, WO 2010/010157, WO 2010/010150, WO 2009/135581, WO 2009/132986, WO 2009/130496, WO 2009/131669, WO 2009/117109, WO 2009/108332, WO 2009/102460, WO 2009/102428, WO 2009/094169, WO 2009/090239, WO 2009/088997, WO 2009/075835, WO 2009/061498, WO 2009/059666, WO 2008/157752, WO 2008/156601, WO 2008/106128, WO 2008/088540, WO 2008/074384, WO 2008/069313, WO 2008/052638, WO 2009/056881, WO 2008/046758, WO 2008/024497, WO 2008/012532, WO 2008/011453, WO 2008/003611, WO 2007/137066, WO 2007/130898, WO 2007/127688, WO 2007/103719, WO 2007/101270, WO 2007/092435, WO 2007/089683, WO 2007/084314, WO 2007/068330, WO 2007/067504, WO 2007/057768, WO 2007/038138, WO 2007/025880, WO 2007/003521, WO 2006/134467, WO 2006/132436, WO 2006/055752, WO 2006/053024, WO 2006/048750, WO 2006/034804, WO 2006/020598, US 2006-0025445, WO 2006/000371, WO 2006/002361, WO 2006/012226, WO 2006/002349, WO 2006/012173, WO 2006/012227, WO 2006/002350, WO 2005/110992, WO 2005/108359, WO 2009/026422, WO 2005/097759, US 8,178,546, WO 2005/060963, WO 2005/046685, WO 2005/047250, WO 2004/112784, WO 2004/112779, WO 2004/112781, WO 2004/112782, WO 2004/112783, WO 2004/112785, WO 2004/113310, WO 2004/058730, WO 2004/041264, WO 200/4033427, WO 2004/011410, WO 2003/104208, WO 2003/075660, WO 2003/065983, WO 2003/043999, WO 2003/044000 and WO 01/90091.

Therefore, there is a need in the art to develop further inhibitors 11-beta-hydroxysteroid dehydrogenase type 1.

### SUMMARY OF THE INVENTION

The inventors have surprisingly found that compounds of formula (I) are inhibitors of the 11-beta-hydroxysteroid dehydrogenase type 1 (11-beta-HSD1).

### DETAILED DESCRIPTION OF THE INVENTION

In a first aspect, the invention relates to compounds of formula (I): 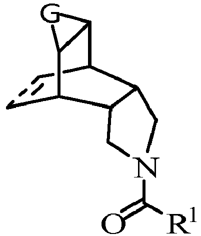 wherein
R¹ is selected from the group consisting of:
   a) C₃₋₈ cycloalkyl, optionally substituted with 1 to 3 groups selected from halogen atoms and C₁₋₄ alkyl;
   b) C₃₋₈ cycloalkenyl, optionally substituted with 1 to 3 groups selected from halogen atoms and C₁₋₄ alkyl;
   c) C₆₋₁₀ aromatic group optionally substituted with 1 to 3 groups selected from halogen atoms, CN, CF₃, NH₂, OH, C₁₋₄ alkyl and C₁₋₄ alkoxy;
   d) C₅₋₁₀ heteroaromatic group optionally substituted with 1 to 3 groups selected from halogen atoms, CN, CF₃, NHR³, C₁₋₄ alkyl, C₁₋₄ alkoxy, C₅₋₁₀ heteroaryl (optionally substituted with 1 to 3 groups selected from halogen atoms, CN, CF₃, NH₂, OH, C₁₋₄ alkyl and C₁₋₄ alkoxy), phenyl (optionally substituted with one or two groups selected from halogen atoms, CN, OH, CF₃, C₁₋₄ alkyl and C₁₋₄ alkoxy) and C₅₋₇ heterocyclyl (optionally substituted with a group selected from -COR⁴, -COOR⁴, -CN, -CONHR⁴, -CONH₂ and phenyl which is optionally substituted with one or two groups selected from halogen atoms, CONH₂, CN, OH, CF₃, C₁₋₄ alkyl and C₁₋₄ alkoxy), wherein the C₅₋₇ heterocyclyl group comprises a nitrogen atom forming part of the heterocycle and is attached to the C₅₋₁₀ heteroaromatic group through said nitrogen atom;
   e) C₃₋₈ heterocyclyl optionally substituted with a group selected from C₁₋₄ alkyl, C₃₋₈ cycloalkyl and -COR⁵; and
   f) -C(CH₃)₂-R²;
G is a rest selected from the group consisting of -CH₂-, -CH₂-CH₂- and -CH=CH-;
R² is selected from the group consisting of hydrogen atom and C₁₋₃ alkyl
R³ is selected from the group consisting of C₁₋₃ alkyl and C₁₋₃ hydroxyalkyl
R⁴ is selected from the group consisting of C₁₋₆ alkyl and phenyl
R⁵ is a group C₁₋₆ alkyl
the bond represented by -̅-̅-̅-̅ is either a single bond or a double bond; and pharmaceutically stable salts thereof for use in the treatment or prevention of a disease or conditions susceptible of improvement by inhibition of 11β-hydroxysteroid dehydrogenase type 1 (11β-HD1), preferably a disease or condition selected from the group consisting of obesity, insulin resistance, metabolic syndrome, cognitive disfunction associated to Alzheimer's disease, HIV-associated dementia, Creutzfeld-Jakob disease, Parkinson's disease, Huntington's disease, vascular inflammation, atherosclerosis, diabetes type 2, cardiovascular disease, glaucoma, osteoporosis, myocardial infarction and alcohol abuse treatment.
The compounds of formula (I) defined in the first aspect are new.

Thus, in a second aspect, the invention relates to new compounds of formula (I): 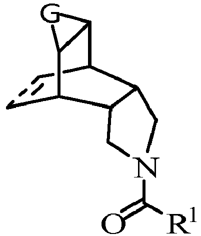 wherein
R¹ is selected from the group consisting of:
   a) C₃₋₈ cycloalkyl, optionally substituted with 1 to 3 groups selected from halogen atoms and C₁₋₄ alkyl;
   b) C₃₋₈ cycloalkenyl, optionally substituted with 1 to 3 groups selected from halogen atoms and C₁₋₄ alkyl;
   c) C₆₋₁₀ aromatic group optionally substituted with 1 to 3 groups selected from halogen atoms, CN, CF₃, NH₂, OH, C₁₋₄ alkyl and C₁₋₄ alkoxy;
   d) C₅₋₁₀ heteroaromatic group optionally substituted with 1 to 3 groups selected from halogen atoms, CN, CF₃, NHR³, C₁₋₄ alkyl, C₁₋₄ alkoxy, C₅₋₁₀ heteroaryl (optionally substituted with 1 to 3 groups selected from halogen atoms, CN, CF₃, NH₂, OH, C₁₋₄ alkyl and C₁₋₄ alkoxy), phenyl (optionally substituted with one or two groups selected from halogen atoms, CN, OH, CF₃, C₁₋₄ alkyl and C₁₋₄ alkoxy) and C₅₋₇ heterocyclyl (optionally substituted with a group selected from -COR⁴, -COOR⁴, -CN, -CONHR⁴, -CONH₂ and phenyl which is optionally substituted with one or two groups selected from halogen atoms, CONH₂, CN, OH, CF₃, C₁₋₄ alkyl and C₁₋₄ alkoxy), wherein the C₅₋₇ heterocyclyl group comprises a nitrogen atom forming part of the heterocycle and is attached to the C₅₋₁₀ heteroaromatic group through said nitrogen atom;
   e) C₃₋₈ heterocyclyl optionally substituted with a group selected from C₁₋₄ alkyl, C₃₋₈ cycloalkyl and -COR⁵; and
   f) -C(CH₃)₂-R²;
G is a rest selected from the group consisting of -CH₂-, -CH₂-CH₂- and -CH=CH-;
R² is selected from the group consisting of hydrogen atom and C₁₋₃ alkyl
R³ is selected from the group consisting of C₁₋₃ alkyl and C₁₋₃ hydroxyalkyl
R⁴ is selected from the group consisting of C₁₋₆ alkyl and phenyl
R⁵ is a group C₁₋₆ alkyl
the bond represented by -̅-̅-̅-̅ is either a single bond or a double bond;
and pharmaceutically stable salts thereof.

In one embodiment of the first aspect and second aspects of the present invention in the compounds of formula (I) the rest 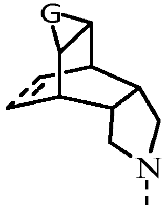 is selected from the group consisting of rests (A) to (D):

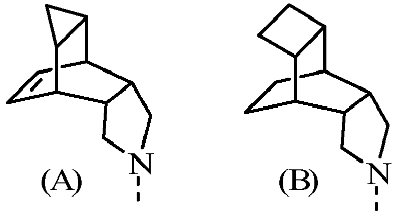

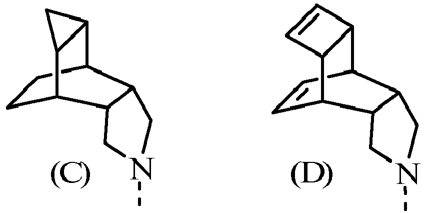

In another embodiment of the first aspect and second aspects of the present invention in the compounds of formula (I) the rest 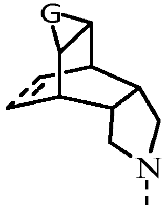 is selected from the group consisting of rests (A) and (C):

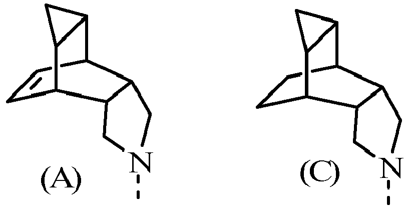

In another embodiment of the first and second aspects of the present invention in the compounds of formula (I) G is a rest -CH₂-.

In another embodiment of the first and second aspects of the present invention in the compounds of formula (I) the bond represented by -̅-̅-̅-̅ is a double bond.

In another embodiment of the first and second aspects of the present invention in the compounds of formula (I) R¹ is a C₃₋₈ cycloalkyl, optionally substituted with 1 to 3 groups selected from halogen atoms and C₁₋₄ alkyl.

In another embodiment of the first and second aspects of the present invention in the compounds of formula (I) R¹ is a C₃₋₈ cycloalkenyl, optionally substituted with 1 to 3 groups selected from halogen atoms and C₁₋₄ alkyl.

In another embodiment of the first and second aspects of the present invention in the compounds of formula (I) R¹ is a C₆₋₁₀ aromatic group optionally substituted with 1 to 3 groups selected from halogen atoms, CN, CF₃, NH₂, OH, C₁₋₄ alkyl and C₁₋₄ alkoxy.

In another embodiment of the first and second aspects of the present invention in the compounds of formula (I) R¹ is a C₅₋₁₀ heteroaromatic group optionally substituted with 1 to 2 groups selected from halogen atoms, CN, CF₃, NHR³, C₁₋₄ alkyl, C₁₋₄ alkoxy, C₅₋₁₀ heteroaryl (optionally substituted with 1 to 3 groups selected from halogen atoms, CN, CF₃, NH₂, OH, C₁₋₄ alkyl and C₁₋₄ alkoxy), phenyl (optionally substituted with a group selected from halogen atoms, CN, OH, CF₃, C₁₋₄ alkyl and C₁₋₄ alkoxy) and C₅₋₇ heterocyclyl (optionally substituted with a group selected from -COR⁴,-COOR⁴, -CN, -CONHR⁴, -CONH₂ and phenyl which is optionally substituted with one or two groups selected from halogen atoms, CONH₂, CN, OH, CF₃, C₁₋₄ alkyl and C₁₋₄ alkoxy), wherein the C₅₋₇ heterocyclyl group comprises a nitrogen atom forming part of the heterocycle and is attached to the C₅₋₁₀ heteroaromatic group through said nitrogen atom, wherein R³ is selected from the group consisting of C₁₋₃ alkyl and C₁₋₃ hydroxyalkyl and R⁴ is selected from the group consisting of C₁₋₆ alkyl and phenyl.

In another embodiment of the first and second aspects of the present invention in the compounds of formula (I) R¹ is a C₃₋₈ heterocyclyl optionally substituted with a group selected from C₁₋₄ alkyl, C₃₋₈ cycloalkyl and COR⁵ wherein R⁵ is a group C₁₋₆ alkyl.

In another embodiment of the first and second aspects of the present invention in the compounds of formula (I) R¹ is a group -C(CH₃)₂-R², wherein R² is selected from the group consisting of hydrogen atom and C₁₋₃ alkyl.

In another embodiment of the first and second aspects of the present invention in the compounds of formula (I) R⁵ is a group methyl.

### Process for producing the compounds of formula (I)

In a third aspect, the invention relates to a process for producing a compound of formula (I): 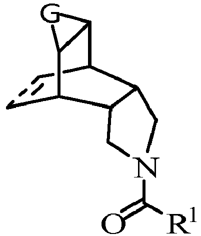 as defined in the first or second aspects of the invention by causing a polycyclic amine of formula (II) or a salt thereof to react with an acid derivative of formula (III).

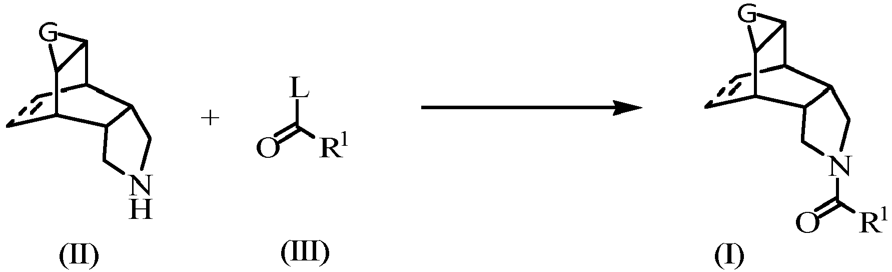

The group L in the compound of formula (III) is selected from the group consisting of halogen atoms, C₁₋₄-alkoxy, OH, and a group of formula -O-CO-R¹, i.e. the compounds of formula (III) are carboxylic acid halides, carboxylic acid esters, carboxylic acids or carboxylic acid anhydrides.

The reaction of compounds of formula (II) with compounds of formula (III) can be carried out following different methods known by any person skilled in the field of organic synthesis. In a preferred embodiment the group L is OH (i.e. the compounds of formula (III) are carboxylic acids) and the reaction is carried out in a solvent such as ethyl acetate in the presence of 1-ethyl-3-(3-dimethylaminopropyl)carbodiimide (EDC), hydroxybenzotriazole (HOBt) and triethylamine (NEt₃), preferably at room temperature. Alternatively, the group L is Cl (i.e. the compounds of formula (III) are carboxylic acid chlorides) and the reaction is carried out in a solvent such as acetone or dichloromethane in the presence of a base such as triethylamine (NEt₃), preferably at room temperature. In turn, the carboxylic acid chlorides (compounds of formula (III) when the group L is Cl) can be obtained from the corresponding carboxylic acid using a large variety of reagents such as thionyl chloride, sulfuryl chloride, oxalyl chloride, etc. In another embodiment, the group L is a group of formula -O-CO-R¹ (i.e. the compounds of formular (III) are carboxylic acid anhydrides) and the reaction is carried out in a solvent such as dichloromethane in the presence of a base such a triethylamine (NEt₃).

The compounds of the invention wherein the bond represented by -̅-̅-̅-̅ is a single bond (compounds of formula (Ib) may be also obtained by hydrogenation of the compounds of the invention wherein the bond represented by -̅-̅-̅-̅ is a double bond (compounds of formula (Ia) as shown in the scheme below:

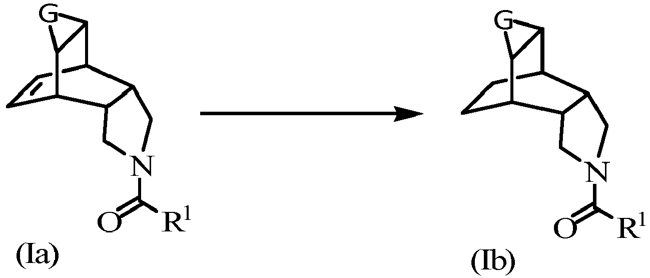

In an embodiment the hydrogenation is carried out in a solvent such as ethanol (or methanol) using hydrogen gas in the presence of a metal catalyst such as Pd/C, preferably at room temperature and at 1 atmosphere of pressure. Alternatively, the reduction can be carried out using other methods known by the skilled person, such as diimide or hydrogen transfer hydrogenation (HCO₂H, Et₃N, Pd).

The compounds of formula (II) are either commercially available or may be obtained by the process detailed in Rey-Carrizo, M et al., J Med Chem. 2014 Jul 10;57(13):5738-47.

Similarly the compounds of formula (III) are either commercially available or may be obtained by processes known to the expert in the field.

When, in the compounds of formula (I), the group R¹ is a C₅₋₁₀ heteroaromatic group substituted with groups selected from NHR³ and C₅₋₇ heterocyclyl (optionally substituted with a group selected from -COR⁴, -COOR⁴, -CN, -CONHR⁴, -CONH₂ and phenyl which is optionally substituted with one or two groups selected from halogen atoms, CONH₂, CN, OH, CF₃, C₁₋₄ alkyl and C₁₋₄ alkoxy), wherein the C₅₋₇ heterocyclyl group comprises a nitrogen atom forming part of the heterocycle and is attached to the C₅₋₁₀ heteroaromatic group through said nitrogen atom; said compounds of formula (Id) may be obtained by reacting a compound having the corresponding C₅₋₁₀ heteroaromatic group substituted by a halogen atom (Ic) with a compound of formula R⁶-H wherein R⁶ represents a group selected from NHR³ and C₅₋₇ heterocyclyl (optionally substituted with a group selected from -COR⁴, -COOR⁴, -CN, -CONHR⁴,-CONH₂ and phenyl which is optionally substituted with one or two groups selected from halogen atoms, CONH₂, CN, OH, CF₃, C₁₋₄ alkyl and C₁₋₄ alkoxy), wherein the C₅₋₇ heterocyclyl group comprises a nitrogen atom forming part of the heterocycle and is attached to the C₅₋₁₀ heteroaromatic group through said nitrogen atom as shown in scheme below:

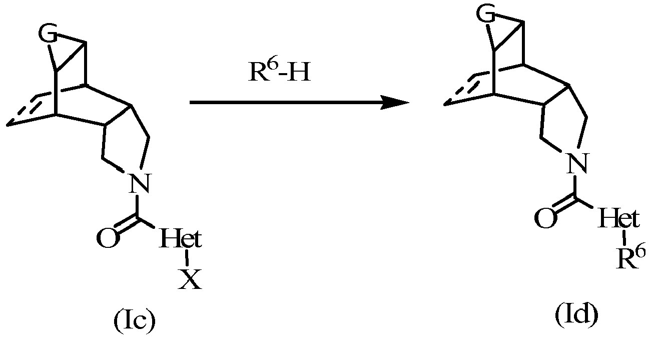

When, in the compounds of formula (I), the group R¹ is a C₅₋₁₀ heteroaromatic group substituted by a group selected from C₅₋₁₀ heteroaryl (which is optionally substituted with 1 to 3 groups selected from halogen atoms, CN, CF₃, NH₂, OH, C₁₋₄ alkyl and C₁₋₄ alkoxy) or by a phenyl group which is optionally substituted with one or two groups selected from halogen atoms, CONH₂, CN, OH, CF₃, C₁₋₄ alkyl and C₁₋₄ alkoxy; said compounds of formula (Ie) may be obtained by reacting a compound having the corresponding C₅₋₁₀ heteroaromatic group substituted by a halogen atom (Ic) with a heteroaryl boronic acid or a phenyl boronic acid in the presence of a catalyst such as Pd(PPh₃)₄ and a base, such as K₂CO₃, wherein the phenyl is optionally substituted with one or two groups selected from halogen atoms, CONH₂, CN, OH, CF₃, C₁₋₄ alkyl and C₁₋₄ alkoxy and the heteroaryl boronic acid is optionally substituted with 1 to 3 groups selected from halogen atoms, CN, CF₃, NH₂, OH, C₁₋₄ alkyl and C₁₋₄ alkoxy as shown in scheme below:

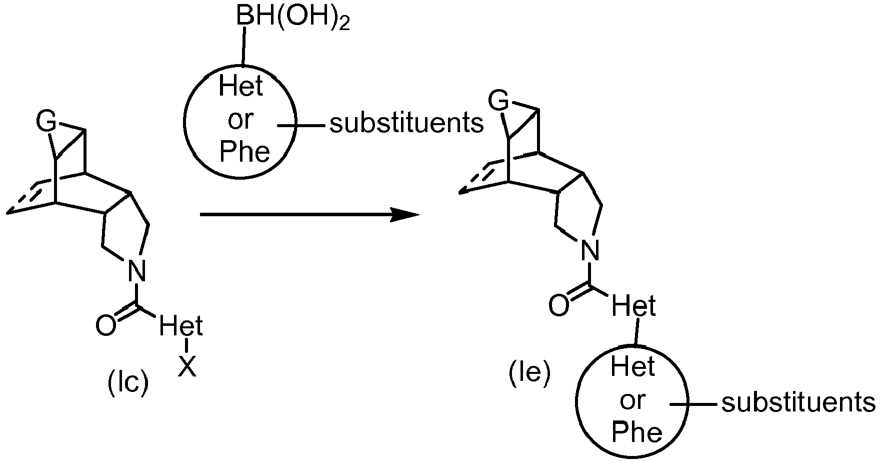

### Medical uses of the compounds of the invention

The compounds of the invention can be used in the treatment or prevention of a disease or condition susceptible of improvement by inhibition of 11β-hydroxysteroid dehydrogenase type 1 (11β-HD1), in particular a disease or condition selected from the group consisting of obesity, insulin resistance, metabolic syndrome, cognitive disfunction associated to Alzheimer's disease, HIV-associated dementia, Creutzfeld-Jakob disease, Parkinson's disease, Huntington's disease, vascular inflammation, atherosclerosis, diabetes type 2, cardiovascular disease, glaucoma, osteoporosis, myocardial infarction and alcohol abuse treatment.

### Definitions

The term "alkyl", as used herein, refers to; is used to designate linear or branched hydrocarbon fully saturated radicals (CₙH₂ₙ₊₁). The indication C_{x-y}. preceding the term "alkyl" is used to indicate that the alkyl may have from x to y carbon atoms. Examples of alkyls include methyl, ethyl, n-propyl, i-propyl, n-butyl, sec-butyl, tert-butyl, n-pentyl, 1-methyl-butyl, 2-methyl-butyl, isopentyl, 1-ethylpropyl, 1,1-dimethylpropyl, 1,2-dimethylpropyl, n-hexyl, 1-ethylbutyl, 2-ethylbutyl, 1,1-dimethylbutyl, 1,2-dimethylbutyl, 1,3-dimethylbutyl, 2,2-dimethylbutyl, 2,3-dimethylbutyl, 2-methylpentyl and 3-methylpentyl radicals. In a preferred embodiment said alkyl groups have 1 to 4 carbon atoms or 1 to 3 carbon atoms.

The term "alkoxy", as used herein, refers to radicals which contain a linear or branched alkyl group linked to an oxygen atom (CₙH₂ₙ₊₁-O-). The indication C_{x-y}. preceding the term "alkoxy" is used to indicate that the alkoxy may have from x to y carbon atoms. Preferred alkoxy radicals include methoxy, ethoxy, n-propoxy, i-propoxy, n-butoxy, sec-butoxy and t-butoxy.

The term "hydroxyalkyl", as used herein, refers to radicals which contain a linear or branched alkyl group in which one hydrogen atom has been replaced by a hydroxyl (-OH) group. The indication C_{x-y}. preceding the term "hydroxyalkyl" is used to indicate that the hydroxyalkyl may have from x to y carbon atoms. Preferred hydroxyalkyl radicals include hydroxymethyl, hydroxyethyl, n-hydroxypropyl, i-hydroxypropyl, n-hydroxybutyl, sec-hydroxybutyl and t-hydroxybutyl.

The term "cycloalkyl", as used herein, refers to hydrocarbon fully saturated cyclic groups atoms (CₙH₂ₙ₋₁). The indication C_{x-y}. preceding the term "cycloalkyl" is used to indicate that the cycloalkyl may have from x to y carbon atoms Said cycloalkyl groups may have a single cyclic ring or multiple condensed rings. Such cycloalkyl groups include, by way of example, single ring structures such as cyclopropyl, cyclobutyl, cyclopentyl, cyclooctyl, and the like, or multiple ring structures such as adamantanyl, bicyclo[2.2.1]heptane,1,3,3-trimethylbicyclo[2.2.1]hept-2-yl, (2,3,3-trimethylbicyclo [2.2.1]hept-2-yl), or carbocyclic groups fused with an aryl group, for example indane, and the like.

The term "cycloalkenyl", as used herein, refers to hydrocarbon partially unsaturated cyclic groups atoms comprising only single or double bonds between the carbon atoms of the cycle. A hydrocarbon cyclic group is considered to be partially unsaturated when it has a number of unsaturations which is lower than the maximum possible number of unsaturations admitted by said hydrocarbon cycle. The indication C_{x-y}. preceding the term "cycloalkenyl" is used to indicate that the cycloalkenyl may have from x to y carbon atoms. Said cycloalkenyl groups may have a single cyclic ring or multiple condensed rings. Such cycloalkenyl groups include, by way of example, single ring structures such as cyclopropenyl, cyclobutenyl, cyclopentenyl, cyclooctenyl, and the like, or multiple ring structures.

The term "halogen atom", as used herein, is used to designate an atom selected from the group consisting of chlorine, fluorine, bromine or iodine atoms typically a fluorine, chlorine or bromine atom. The term halo when used as a prefix has the same meaning.

The term "aromatic group", as used herein, refers to a cyclic aromatic group wherein the cycle is made exclusively of carbon atoms. According to the IUPAC a cyclically conjugated group is said to be aromatic when it has a stability (due to delocalization) significantly greater than that of a hypothetical localized structure. The indication C_{x-y}. preceding the term "aromatic group" is used to indicate that said group may have from x to y carbon atoms. Examples of aromatic groups are benzene, naphthalene, anthracene and phenanthrene.

The terms "heterocyclyl" and "heterocycle", as used herein, refer to nonaromatic, saturated or unsaturated ring systems comprising carbon atoms and one or more heteroatoms selected from the group consisting of oxygen, nitrogen and sulphur. The indication C_{x-y}. preceding the term "heterocyclyl" is used to indicate that the heterocyclyl may have from x to y carbon atoms. Said heterocyclyl groups may have a single cyclic ring or multiple condensed rings. Such heterocyclyl groups include, by way of example, single ring structures such as azetidine, oxetane, thietane, diazetidine, dioxetane, tetrahydrofurane, pyran, dihydropyran, tetrahydropyran, pyrrolidine, thiolane, imidazolidine, pyrazolidine, oxazolidine, isoxazolidine, thiazolidine, isothiazolidine, dioxolane, dithiolane, piperidine, oxane, thiane, piperazine, morpholine, thiomorpholine, dioxane, dithiane, azepane, oxepane and the like or multiple ring structures.

The term "optionally substituted", as used herein, refers to the fact that a group or rest may have one or more of its hydrogen atoms replaced (substituted) by an atom or group different from hydrogen.

The term "heteroaromatic group" or heteroaryl, as used herein indistinctively, refers to a cyclic aromatic group wherein the cycle is made of carbon atoms and at least one atom selected from the group consisting of oxygen, nitrogen and sulphur. According to the IUPAC a cyclically conjugated group is said to be aromatic when it has a stability (due to delocalization) significantly greater than that of a hypothetical localized structure. The indication Cx-y. preceding the term "heteroaromatic group" is used to indicate that said group may have from x to y carbon atoms. Examples of heteraromatic groups are pyridine, pyrrol, furan, thiophene, pyrazine, pyrimidine, pyridazine, imidazole, pyrazole, oxazole, thiophene, benzimidazole, quinolone, isoquinoline, quinoxaline, quinazoline, cinnoline, phathalazine, imidazopyridine, pyrazolopyridine, indazole, benzooxazole, benzofuran, isobenzofuran, benzothiophene, indol and isoindol.

The term "acetyl", as used herein, refers to the group CH₃-C(=O)-;

The term "pharmaceutically acceptable" relates to molecular entities and compositions being physiologically tolerable and normally not causing an allergic reaction or similar adverse reaction, such as gastric discomfort, dizziness and the like, when they are administered to a human being. Preferably, as used in this description, the term "pharmaceutically acceptable" means approved by a governmental regulatory agency or listed in the US pharmacopoeia or another generally recognized pharmacopoeia for use in animals, and more particularly in humans. When applied to salts the term "pharmaceutically acceptable" encompasses salts with a pharmaceutically acceptable acid or base. Pharmaceutically acceptable acids include inorganic acids, such as hydrochloric, sulphuric, phosphoric, diphosphoric, hydrobromic, hydroiodic and nitrate acids, and organic acids, such as citric, maleic, malic, mandelic, ascorbic, oxalic, succinic, tartaric, acetic, methanesulfonic, ethanesulfonic, benzenesulfonic and p-toluenosulfonic acids. Pharmaceutically acceptable bases include hydroxides of alkali metals (e.g. sodium or potassium), alkaline-earth metals (for example, calcium or magnesium) and organic bases (for example, alkylamines, arylalkyilamines and heterocyclic amines).

Other preferred salts according to the invention are quaternary ammonium compounds in which an equivalent of an anion (X⁻) is associated with the positive charge of the N atom. X⁻ may be an anion of diverse mineral acids such as for example, chloride, bromide, iodide, sulfate, nitrate, phosphate, or an anion of an organic acid, such as acetate, maleate, fumarate, citrate, oxalate, succinate, tartrate, malate, mandelate, trifluoracetate, methanesulfonate and p-toluenesulfonate. X⁻ is preferably an anion selected from chloride, bromide, iodide, sulfate, nitrate, acetate, maleate, oxalate, succinate and trifluoracetate. More preferably X⁻ is chloride, bromide, trifluoracetate or methanesulfonate.

The term "prevention", as used herein, refers to the administration of the compounds of the invention in an initial or early stage of a disease, or to also prevent its onset.

The term "treatment" is used to designate the administration of the compounds of the invention to control disorder progression before or after the clinical signs had appeared. By control of the disorder progression it is meant to designate beneficial or desired clinical results including, but not limited to, reduction of symptoms, reduction of the length of the disorder, stabilization pathological state (specifically avoidance of further deterioration), delay in the disorder's progression, improvement of the pathological state and remission (both partial and total). In a particular embodiment of the invention the compounds of the invention are used to control the disorder progression once at least one of the disorder's clinical signs has appeared.

The term "medicament", as used herein, refers to a pharmaceutical composition comprising a compound of formula (I). The medicament may be administered by any suitable route. It is prepared by conventional means with pharmaceutically acceptable excipients.

The term "subject", as used herein, refers to any animal or human that is suffering from one of the diseases disclosed above. Preferably, the subject is a mammal. The term "mammal", as used herein, refers to any mammalian species, including but not being limited to domestic and farm animals (cows, horses, pigs, sheep, goats, dogs, cats or rodents), primates, and humans. Preferably, the mammal is selected from a human being. In the context of the present invention, the mammal is suffering from a disease selected from the group consisting of obesity, insulin resistance, metabolic syndrome, cognitive disfunction associated to Alzheimer's disease, HIV-associated dementia, Creutzfeld-Jakob disease, Parkinson's disease, Huntington's disease, vascular inflammation, atherosclerosis, diabetes type 2, cardiovascular disease, glaucoma, osteoporosis, myocardial infarction and alcohol abuse treatment, or in risk of suffering from one of said diseases.

The invention is described below by means of several examples which do not limit, but rather illustrate the invention.

### Examples

### General

Reagents, solvents and starting products were acquired from commercial sources. The term "concentration" refers to the vacuum evaporation using a Büchi rotavapor. When indicated, the reaction products were purified by "flash" chromatography on silica gel (35-70 µm) with the indicated solvent system. The melting points were measured in a Büchi B-540 or in a MFB 59510M Gallenkamp instruments. IR spectra were run on FTIR Perkin-Elmer Spectrum RX I spectrophotometer using potassium bromide (KBr) pellets or attenuated total reflectance (ATR) technique. Absorption values are expressed as wavenumbers (cm⁻¹); only significant absorption bands are given. The accurate mass analyses were carried out using a LC/MSD-TOF spectrophotometer. The elemental analyses were carried out in a Flash 1112 series Thermofinnigan elemental microanalyzator (A5) to determine C, H, and N.

### Example 1: cyclohexyl((4R,4aR,5aS,6S,6aS)-3,3a,4,4a,5,5a,6,6a-octahydro-4,6-etheno-cyclopropa[f]isoindol-2(1H)-yl)methanone

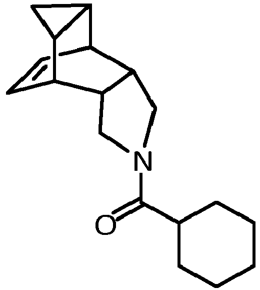

To a solution of 4-azatetracyclo[5.3.2.0^{2,6}.0^{8,10}]dodec-11-ene hydrochloride (2.057 g, 10.63 mmol) in EtOAc (150 mL) were added cyclohexanecarboxylic acid (1.239 g, 9.67 mmol), HOBt (1.9585 g, 14.50 mmol), EDC (2.2456 g, 14.50 mmol) and triethylamine (5.9 mL, 42.53 mmol). The reaction mixture was stirred at room temperature overnight. To the resulting suspension was then added water (150 mL) and the phases were separated. The organic phase was washed with saturated aqueous NaHCO₃ solution (150 mL) and brine (150 mL), dried over anh. Na₂SO₄ and filtered. Evaporation in *vacuo* of the organics gave the title compound as a yellowish solid (2.4346 g, 94.3% yield). The analytical sample was obtained by crystallization from hot EtOAc (2.0392 g), m.p. 96-97 °C. IR (ATR) v: 560, 570, 587, 696, 718, 741, 767, 812, 829, 847, 894, 915, 942, 963, 991, 1036, 1089, 1134, 1167, 1209, 1217, 1242, 1272, 1299, 1361, 1380, 1432, 1624, 2849, 2925, 3002, 3040 cm⁻¹. Elemental analysis: Calculated for C₁₈H₂₅NO: C 79.66%, H 9.29%, N 5.16%. Found: C 79.64%, H 9.24%, N 5.21%.

### Example 2: cyclohexyl((4R,4aR,5aS,6S,6aS)-octahydro-4,6-ethanocyclopropa[f]iso-indol-2(1H)-yl)methanone

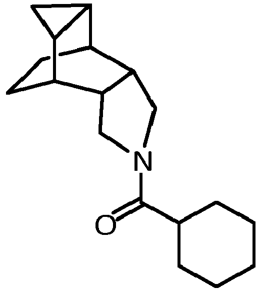

A solution of the compound from example 1 (500 mg) and Pd/C (100 mg) in absolute EtOH (85 mL) was hydrogenated at 1 atm for 72 hours. The dark solution was filtered and concentrated in vacuo to give the title compound as a white solid (426 mg, 82.6% yield), m. p. 74-75 °C. IR (ATR) v: 651, 679, 707, 729, 748, 789, 805, 826, 839, 865, 885, 950, 961, 988, 1016, 1030, 1082, 1113, 1133, 1171, 1205, 1213, 1234, 1264, 1295, 1325, 1346, 1358, 1427, 1471, 1486, 1623, 2846, 2897, 2928, 3009, cm⁻¹. Elemental analysis: Calculated for C₁₈H₂₇NO: C 79.07%, H 9.95%, N 5.12%. Found: C 79.15%, H 9.88%, N 5.29%.

### Example 3: cyclohexyl((4R,4aR,6aS,7S,7aS)-1,3,3a,4,4a,6a,7,7a-octahydro-2H-4,7-ethenocyclobuta[f]isoindol-2-yl)methanone

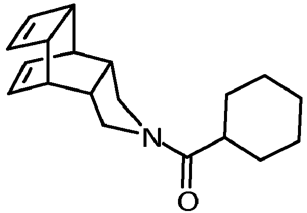

To a solution of 4-azatetracyclo[5.4.2.0^{2,6}.0^{8,11}]trideca-9,12-diene hydrochloride (139 mg, 0.66 mmol) in EtOAc (6 mL) were added cyclohexanecarboxylic acid (77 mg, 0.60 mmol), HOBt (122 mg, 0.90 mmol), EDC (139 mg, 0.90 mmol) and triethylamine (0.4 mL, 2.64 mmol). The reaction mixture was stirred at room temperature overnight. To the resulting suspension was then added water (10 mL) and the phases were separated. The organic phase was washed with saturated aqueous NaHCO₃ solution (10 mL) and brine (10 mL), dried over anh. Na₂SO₄ and filtered. Evaporation in *vacuo* of the organics gave the title compound as a yellowish solid (151 mg, 80.0% yield). The analytical sample was obtained by crystallization from hot EtOAc (97 mg), m. p. 120-121 °C. IR (ATR) v: 730, 760 788, 808, 967, 994, 1173, 1187, 1214, 1235, 1294, 1361, 1442, 1463, 1617, 2860, 2901, 2922 cm⁻¹. Elemental analysis: Calculated for C₁₉H₂₅NO: C 80.52%, H 8.89%, N 4.94%. Found: C 80.31%, H 8.81%, N 4.97%.

### Example 4: cyclohexyl((4R,4aR,6aS,7S,7aS)-decahydro-2H-4,7-ethanocyclo-buta[f]isoindol-2-yl)methanone

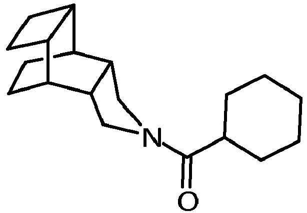

To a solution of 4-azatetracyclo[5.4.2.0^{2,6}.0^{8,11}]tridecane hydrochloride (235 mg, 1.10 mmol) in EtOAc (10 mL) were added cyclohexanecarboxylic acid (128 mg, 1.0 mmol), HOBt (203 mg, 1.50 mmol), EDC (232 mg, 1.50 mmol) and triethylamine (0.6 mL, 4.40 mmol). The reaction mixture was stirred at room temperature overnight. To the resulting suspension was then added water (10 mL) and the phases were separated. The organic phase was washed with saturated aqueous NaHCO₃ solution (10 mL) and brine (10 mL), dried over anh. Na₂SO₄ and filtered. Evaporation in *vacuo* of the organics gave the title compound as a yellowish solid (253 mg, 80.0% yield). The analytical sample was obtained by crystallization from hot EtOAc (110 mg), m. p. 115-116 °C. IR (ATR) v: 658, 731, 887, 988, 1133, 1172, 1204, 1236, 1357, 1434, 1440, 1621, 2908, 2925 cm⁻¹. Elemental analysis: Calculated for C₁₉H₂₉NO: C 79.39%, H 10.17%, N 4.87%. Found: C 79.20%, H 10.30%, N 4.72%.

### Example 5: ((4R,4aR,5aS,6S,6aS)-3,3a,4,4a,5,5a,6,6a-octahydro-4,6-ethenocyclopropa[f]isoindol-2(1H)-yl)(phenyl)methanone

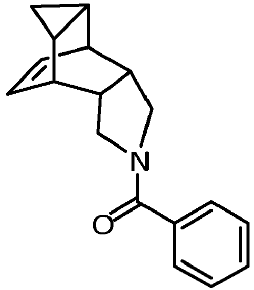

To a solution of 4-azapentacyclo[5.3.2.0^{2,6}.0^{8,10}]dodec-11-ene hydrochloride (400 mg, 2.07 mmol) in EtOAc (20 mL) were added benzoic acid (230 mg, 1.88 mmol), HOBt (381 mg, 2.82 mmol), EDC (437 g, 2.82mmol) and triethylamine (1.2 mL, 8.27 mmol). The reaction mixture was stirred at room temperature overnight. To the resulting suspension was then added water (20 mL) and the phases were separated. The organic phase was washed with saturated aqueous NaHCO₃ solution (20 mL) and brine (20 mL), dried over anh. Na₂SO₄ and filtered. Evaporation in *vacuo* of the organics gave an orange oil (479 mg, 95.9% yield). Column chromatography (Hexane/Ethyl acetate mixture) gave the title compound as a white solid (385 mg), m. p. 65-66 °C. IR (ATR) v: 660, 700, 715, 763, 794, 814, 847, 986, 1029, 1135, 1170, 1231, 1378, 1423, 1572, 1618, 2845, 2865, 2921, 2946 cm⁻¹. Elemental analysis: Calculated for C₁₈H₁₉NO: C 81.47%, H 7.22%, N 5.28%. Found: C 81.52%, H 7.34%, N 5.25%.

### Example 6: ((4R,4aR,5aS,6S,6aS)-3,3a,4,4a,5,5a,6,6a-octahydro-4,6-ethenocyclopropa[f] iso indol-2(1H)-yl)(pyridin-2-yl)methanone

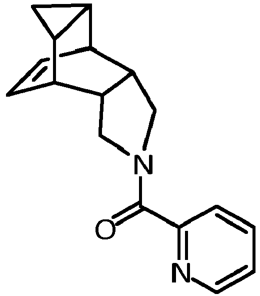

To a solution of 4-azapentacyclo[5.3.2.0^{2,6}.0^{8,10}]dodec-11-ene hydrochloride (400 mg, 2.07 mmol) in EtOAc (20 mL) were added piridin-2-carboxylic acid (231 mg, 1.88 mmol), HOBt (381 mg, 2.82 mmol), EDC (437 mg, 2.82 mmol) and triethylamine (1.2 mL, 8.27 mmol). The reaction mixture was stirred at room temperature overnight. To the resulting suspension was then added water (20 mL) and the phases were separated. The organic phase was washed with saturated aqueous NaHCO₃ solution (20 mL) and brine (20 mL), dried over anh. Na₂SO₄ and filtered. Evaporation in *vacuo* of the organics gave the title compound as an orange oil (466 mg, 93.1% yield). Column chromatography (Hexane/Ethyl acetate mixture) gave an analytical sample as a white solid (326 mg), m. p. 110-111 °C. IR (ATR) v: 682, 720, 753, 796, 814, 844, 912, 988, 1041, 1082, 1142, 1165, 1201, 1226, 1269, 1294, 1302, 1340, 1378, 1400, 1441, 1474, 1562, 1585, 1618, 2850, 2870, 2926, 3007, 3048 cm⁻¹. Elemental analysis: Calculated for C₁₇H₁₈N₂O: C 76.66%, H 6.81%, N 10.52%. Found: C 76.47%, H 7.01%, N 10.21%.

### Example 7: 2,2-dimethyl-1-((4R,4aR,5aS,6S,6aS)-3,3a,4,4a,5,5a,6,6a-octahydro-4,6-ethenocyclopropa[f]isoindol-2(1H)-yl)propan-1-one

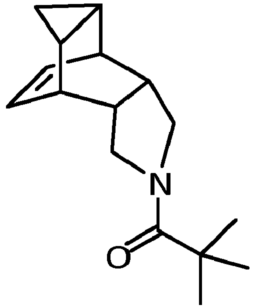

To a solution of 4-azapentacyclo[5.3.2.0^{2,6}.0^{8,10}]dodec-11-ene hydrochloride (200 mg, 1.03 mmol) in EtOAc (10 mL) were added pivalic acid (105 mg, 0.94 mmol), HOBt (190mg, 1.41 mmol), EDC (218 mg, 1.41 mmol) and triethylamine (0.6 mL, 4.14 mmol). The reaction mixture was stirred at room temperature overnight. To the resulting suspension was then added water (10 mL) and the phases were separated. The organic phase was washed with saturated aqueous NaHCO₃ solution (10 mL) and brine (10 mL), dried over anh. Na₂SO₄ and filtered. Evaporation in *vacuo* of the organics gave the title compound as a yellowish solid (216 mg, 93.5% yield). The analytical sample was obtained by crystallization from hot EtOAc (69 mg), m. p. 91-92 °C. IR (ATR) v: 720, 756, 766, 809, 829, 849, 912, 943, 988, 1036, 1069, 1094, 1165, 1193, 1239, 1274, 1340, 1362, 1380, 1405, 1461, 1476, 1507, 1610, 2870, 2896, 2936, 2951, 2992 cm⁻¹. Elemental analysis: Calculated for C₁₆H₂₃NO: C 78.32%, H 9.45%, N 5.71%. Found: C 78.16%, H 9.52%, N 5.86%.

### Example 8: ((4R,4aR,5aS,6S,6aS)-3,3a,4,4a,5,5a,6,6a-octahydro-4,6-ethenocyclopropa[f]isoindol-2(1H)-yl)(thiophen-2-yl)methanone

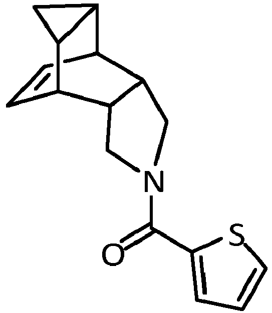

To a solution of 4-azapentacyclo[5.3.2.0^{2,6}.0^{8,10}]dodec-11-ene hydrochloride (200 mg, 1.03 mmol) in EtOAc (10 mL) were added 2-thiophenecarboxylic acid (121 mg, 0.94 mmol), HOBt (190mg, 1.41 mmol), EDC (218 mg, 1.41 mmol) and triethylamine (0.6 mL, 4.14 mmol). The reaction mixture was stirred at room temperature overnight. To the resulting suspension was then added water (10 mL) and the phases were separated. The organic phase was washed with saturated aqueous NaHCO₃ solution (10 mL) and brine (10 mL), dried over anh. Na₂SO₄ and filtered. Evaporation in *vacuo* of the organics gave the title compound as a yellowish solid (219 mg, 85.7% yield). The analytical sample was obtained by crystallization from hot EtOAc (87 mg), m. p. 104-105 °C. IR (ATR) v: 667, 703, 720, 738, 786, 814, 849, 890, 915, 950, 1008, 1031, 1057, 1087, 1132, 1239, 1254, 1279, 1312, 1352, 1380, 1403, 1431, 1519, 1580, 1598, 2921, 2936, 3002, 3037 cm⁻¹. Elemental analysis: Calculated for C₁₆H₁₇NOS: C 70.81%, H 6.31%, N 5.16%. Found: C 70.70%, H 6.28%, N 5.12%.

### Example 9: (4-amino-3,5-dichlorophenyl)((4R,4aR,5aS,6S,6aS)-3,3a,4,4a,5,5a,6,6a-octahydro-4,6-ethenocyclopropa[f]isoindol-2(1H)-yl)methanone

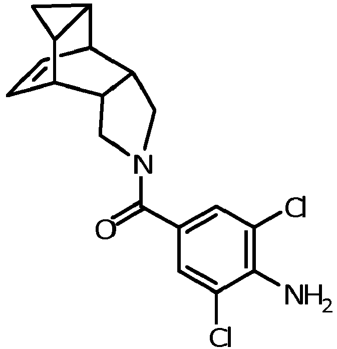

To a solution of 4-azapentacyclo[5.3.2.0^{2,6}.0^{8,10}]dodec-11-ene hydrochloride (200 mg, 1.03 mmol) in EtOAc (10 mL) were added 3,5-dichloro-4-aminobenzoic acid (194 mg, 0.94 mmol), HOBt (190mg, 1.41 mmol), EDC (218 mg, 1.41 mmol) and triethylamine (0.6 mL, 4.14 mmol). The reaction mixture was stirred at room temperature overnight. To the resulting suspension was then added water (10 mL) and the phases were separated. The organic phase was washed with saturated aqueous NaHCO₃ solution (10 mL) and brine (10 mL), dried over anh. Na₂SO₄ and filtered. Evaporation in *vacuo* of the organics gave the title compounds as a yellowish solid (322 mg, 89.3% yield). The analytical sample was obtained by crystallization from hot EtOAc (89 mg), m. p. 186-187 °C. IR (ATR) v: 680, 718, 743, 763, 783, 809, 844, 864, 892, 915, 955, 991, 1034, 1097, 1173, 1223, 1246, 1297, 1347, 1416, 1469, 1501, 1537, 1595, 2875, 2921, 3194, 3240, 3301, 3458 cm⁻¹. Elemental analysis: Calculated for C₁₈H₁₈Cl₂N₂O: C 71.90%, H 5.20%, N 8.02%. Found: C 72.10%, H 5.20%, N 7.92%

### Example 10: cyclohex-3-en-1-yl((4R,4aR,5aS,6S,6aS)-3,3a,4,4a,5,5a,6,6a-octahydro-4,6-ethenocyclopropa[f]isoindol-2(1H)-yl)methanone

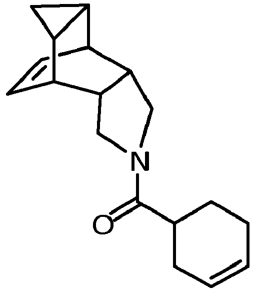

To a solution of 4-azapentacyclo[5.3.2.0^{2,6}.0^{8,10}]dodec-11-ene hydrochloride (200 mg, 1.03 mmol) in EtOAc (10 mL) were added 3-cyclohexene carboxylic acid (119 mg, 0.94 mmol), HOBt (190mg, 1.41 mmol), EDC (218 mg, 1.41 mmol) and triethylamine (0.6 mL, 4.14 mmol). The reaction mixture was stirred at room temperature overnight. To the resulting suspension was then added water (10 mL) and the phases were separated. The organic phase was washed with saturated aqueous NaHCO₃ solution (10 mL) and brine (10 mL), dried over anh. Na₂SO₄ and filtered. Evaporation in *vacuo* of the organics gave the title compound as a yellowish solid (259 mg, quantitative yield). Column chromatography (Hexane/Ethyl acetate mixture) gave the compound as a white solid (199 mg), m. p. 78-79 °C. IR (ATR) v: 682, 710, 763, 816, 839, 854, 887, 915, 940, 981, 1016, 1034, 1087, 1135, 1168, 1203, 1221, 1274, 1292, 1332, 1355, 1380, 1431, 1620, 1651, 2870, 2926, 3022 cm⁻¹. Elemental analysis: Calculated for C₁₈H₂₃NO: C 80.26%, H 8.61%, N 5.20%. Found: C 80.24%, H 8.73%, N 5.19%.

### Example 11: (6-chloropyridin-3-yl)((4R,4aR,5aS,6S,6aS)-3,3a,4,4a,5,5a,6,6a-octahydro-4,6-ethenocyclopropa[f]isoindol-2(1H)-yl)methanone

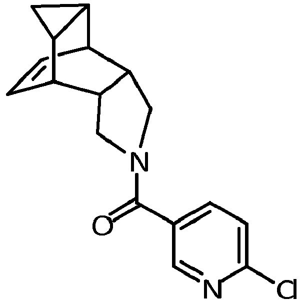

To a solution of 4-azapentacyclo[5.3.2.0^{2,6}.0^{8,10}]dodec-11-ene hydrochloride (500 mg, 2.58 mmol) in EtOAc (25 mL) were added 6-choloronicotinic acid (370 mg, 2.35 mmol), HOBt (477 mg, 3.53 mmol), EDC (547 mg, 3.53 mmol) and triethylamine (1.4 mL, 10.34 mmol). The reaction mixture was stirred at room temperature overnight. To the resulting suspension was then added water (25 mL) and the phases were separated. The organic phase was washed with saturated aqueous NaHCO₃ solution (25 mL) and brine (25 mL), dried over anh. Na₂SO₄ and filtered. Evaporation in *vacuo* of the organics gave the title compound as a yellowish solid (664 mg, 85.6% yield). Column chromatography (Hexane/Ethyl acetate mixture) gave the title compound as a white solid (457 mg), m. p. 101-102 °C. IR (ATR) v: 712, 736, 759, 793, 814, 835, 924, 940, 985, 1030, 1097, 1129, 1156, 1174, 1215, 1239, 1251, 1271, 1283, 1350, 1372, 1430, 1455, 1563, 1583, 1612, 2914, 2948, 3002 cm⁻¹. Elemental analysis: Calculated for C₁₇H₁₇ClN₂O: C 67.88%, H 5.70%, N 9.31%. Found: C 68.14%, H 5.84%, N 9.00%.

### Example 12: (2-chloropyridin-4-yl)((4R,4aR,5aS,6S,6aS)-3,3a,4,4a,5,5a,6,6a-octahydro-4,6-ethenocyclopropa[f]isoindol-2(1H)-yl)methanone

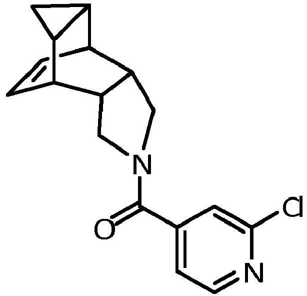

To a solution of 4-azapentacyclo[5.3.2.0^{2,6}.0^{8,10}]dodec-11-ene hydrochloride (500 mg, 2.58 mmol) in EtOAc (25 mL) were added 6-choloronicotinic acid (370 mg, 2.35 mmol), HOBt (477 mg, 3.53 mmol), EDC (547 mg, 3.53 mmol) and triethylamine (1.4 mL, 10.34 mmol). The reaction mixture was stirred at room temperature overnight. To the resulting suspension was then added water (25 mL) and the phases were separated. The organic phase was washed with saturated aqueous NaHCO₃ solution (25 mL) and brine (25 mL), dried over anh. Na₂SO₄ and filtered. Evaporation in *vacuo* of the organics gave the title compound as a yellowish solid (614 mg, 79.1% yield). Column chromatography (Hexane/Ethyl acetate mixture) gave the compound as a white solid (445 mg), m. p. 135-136 °C. IR (ATR) v: 669, 708, 720, 741, 753, 771, 817, 844, 915, 942, 987, 1041, 1091, 1118, 1163, 1176, 1203, 1232, 1245, 1269, 1287, 1342, 1373, 1437, 1464, 1476, 1530, 1593, 1632, 2868, 2932, 3003, 3057 cm⁻¹. Elemental analysis: Calculated for C₁₇H₁₇ClN₂O: C 67.88%, H 5.70%, N 9.31%. Found: C 67.98%, H 5.77%, N9.09%.

### Example 13: ((4R,4aR,5aS,6S,6aS)-3,3a,4,4a,5,5a,6,6a-octahydro-4,6-ethenocyclopropa[f]isoindol-2(1H)-yl)(6-(4-phenylpiperazin-1-yl)pyridin-3-yl)methanone

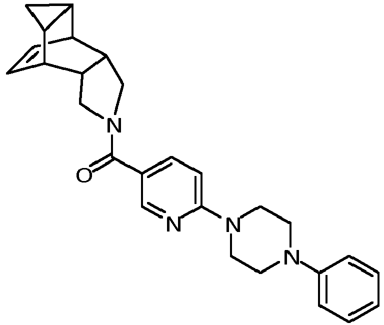

To a solution of the compound from example 11 (100 mg, 0.33 mmol) and 1-phenylpiperazine (60 mg, 0.37 mmol) in DMF (0.5 mL) was added solid K₂CO₃ (82 mg, 0.59 mmol). The resulting suspension was stirred at 90 °C for 48 hours. Water (5 mL) and DCM (5 mL) were added and the phases were separated. The aqueous phase was then extracted with further DCM (2 x 5 mL). The organics were dried over anh. Na₂SO₄, filtered and evaporated in *vacuo* to give a yellowish solid (137 mg). Column chromatography (Hexane/Ethyl acetate mixture) gave the title compound as a white solid (56 mg, 39.4% yield). The analytical sample was obtained by washing with cooled pentane (45 mg), m. p. 90-91 °C. IR (ATR) v: 661, 695, 739, 754, 814, 822, 845, 948, 987, 1013, 1028, 1041, 1095, 1152, 1227, 1310, 1349, 1349, 1395, 1413, 1491, 1594, 1617, 2847, 2919, 2997 cm⁻¹. Accurate mass: Calculated for [C₂₇H₃₁N₄O+H]⁺: 427.2494. Found: 427.2492.

### Example 14: ((4R,4aR,5aS,6S,6aS)-3,3a,4,4a,5,5a,6,6a-octahydro-4,6-ethenocyclopropa[f]isoindol-2(1H)-yl)(6-(4-(4-(trifluoromethyl)phenyl)piperazin-1-yl)pyridin-3-yl)methanone

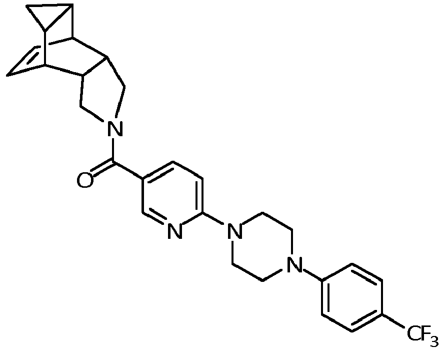

To a solution of the compound from example 11 (100 mg, 0.33 mmol) and 1-(4-trifluoromethylphenyl)piperazine (85 mg, 0.37 mmol) in DMF (0.5 mL) was added solid K₂CO₃ (82 mg, 0.59 mmol). The resulting suspension was stirred at 90 °C for 48 hours. Water (5 mL) and DCM (5 mL) were added and the phases were separated. The aqueous phase was then extracted with further DCM (2 x 5 mL). The organics were dried over anh. Na₂SO₄, filtered and evaporated in *vacuo* to give a yellowish solid (161 mg). Column chromatography (Hexane/Ethyl acetate mixture) gave the title compound as a white solid (52 mg, 31.9% yield). The analytical sample was obtained by washing with cooled pentane (38 mg), m. p. 157-158 °C. IR (ATR) v: 667, 711, 721, 744, 770, 806, 824, 909, 951, 971, 984, 139, 1070, 1106, 1157, 1199, 1230, 1330, 1354, 1390, 1429, 1493, 1522, 1594, 1615, 2847, 2919 cm⁻¹. Accurate mass: Calculated for [C₂₈H₂₉F₂N₄O+H]⁺: 475.2294. Found: 475.2366.

### Example 15: 4-(4-(5-((4R,4aR,5aS,6S,6aS)-1,2,3,3a,4,4a,5,5a,6,6a-decahydro-4,6-ethenocyclopropa[f]isoindole-2-carbonyl)pyridin-2-yl)piperazin-1-yl)benzonitrile

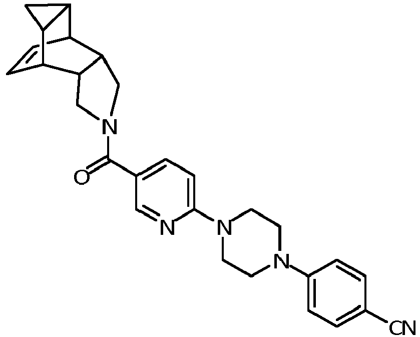

To a solution of the compound from example 11 (100 mg, 0.33 mmol) and 4-piperazinobenzonitrile (69 mg, 0.37 mmol) in DMF (0.5 mL) was added solid K₂CO₃ (82 mg, 0.59 mmol). The resulting suspension was stirred at 90 °C for 48 hours. Water (5 mL) and DCM (5 mL) were added and the phases were separated. The aqueous phase was then extracted with further DCM (2 x 5 mL). The organics were dried over anh. Na₂SO₄, filtered and evaporated in *vacuo* to give a yellowish solid (181 mg). Column chromatography (Hexane/Ethyl acetate mixture) gave the title compound as a white solid (72 mg, 48.3% yield), m. p. 160-161 °C. IR (ATR) v: 656, 692, 713, 742, 773, 811, 912, 951, 1008, 1039, 1176, 1235, 1312, 1392, 1426, 1511, 1537, 1555, 1599, 1648, 1666, 2847, 2925 cm⁻¹. Accurate mass: Calculated for [C₂₈H₃₀N₅O+H]⁺: 452.2444. Found: 452.2445.

### Example 16: (1-methylpiperidin-4-yl)((4R,4aR,5aS,6S,6aS)-3,3a,4,4a,5,5a,6,6a-octahydro-4,6-ethenocyclopropa[f]isoindol-2(1H)-yl)methanone

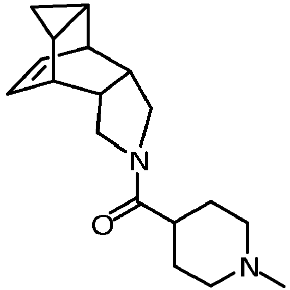

To a solution of 4-azapentacyclo[5.3.2.0^{2,6}.0^{8,10}]dodec-11-ene hydrochloride (200 mg, 1.03 mmol) in EtOAc (10 mL) were added 1-methylpiperidine-4-carboxylic acid (135 mg, 0.94 mmol), HOBt (190mg, 1.41 mmol), EDC (218 mg, 1.41 mmol) and triethylamine (0.6 mL, 4.14 mmol). The reaction mixture was stirred at room temperature for 24 h. To the resulting suspension was then added water (10 mL) and the phases were separated. The organic phase was washed with saturated aqueous NaHCO₃ solution (10 mL) and brine (10 mL), dried over anh. Na₂SO₄ and filtered. Evaporation in *vacuo* of the organics gave a mixture of the product and starting materials as a yellowish solid (129 mg). Column chromatography (Dichloromethane/Methanol) gave the title compound as a white solid (86 mg, 32% yield). The analytical sample was obtained by crystallization from hot EtOAc (50 mg), m. p. 100-101 °C. IR (ATR) v: 718, 767, 819, 835, 850, 876, 915, 987, 1013, 1041, 1067, 1090, 1129, 1150, 1191, 1214, 1250, 1276, 1305, 1359, 1374, 1431, 1447, 1625, 2780, 2857, 2914, 2940, 3328 cm⁻¹. Accurate mass: Calculated for [C₁₈H₂₆N₂O+H]⁺: 287.2118. Found: 287.2113.

### Example 17: 1-(4-((4R,4aR,5aS,6S,6aS)-1,2,3,3a,4,4a,5,5a,6,6a-decahydro-4,6-ethenocyclopropa[f]isoindole-2-carbonyl)piperidin-1-yl)ethan-1-one

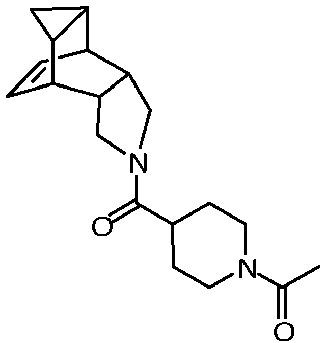

To a solution of 4-azapentacyclo[5.3.2.0^{2,6}.0^{8,10}]dodec-11-ene hydrochloride (200 mg, 1.03 mmol) in EtOAc (10 mL) were added 1-acetyl-4-piperidinecarboxylic acid (161 mg, 0.94 mmol), HOBt (190 mg, 1.41 mmol), EDC (218 mg, 1.41 mmol) and triethylamine (0.6 mL, 4.14 mmol). The reaction mixture was stirred at room temperature for 24 h. To the resulting suspension was then added water (10 mL) and the phases were separated. The organic phase was washed with saturated aqueous NaHCO₃ solution (10 mL) and brine (10 mL), dried over anh. Na₂SO₄ and filtered. Evaporation in *vacuo* of the organics gave a mixture of the product and starting materials (182 mg). Column chromatography (Hexane/Ethyl acetate) gave the title compound as a white solid (134 mg, 45% yield), m. p. 134-135 °C. IR (ATR) v: 605, 703, 762, 814, 829, 920, 956, 977, 997, 1041, 1098, 1116, 1168, 1222, 1271, 1307, 1356, 1426, 1620, 1640, 2852, 2925, 2992 cm⁻¹. Elemental analysis: Calculated for C₁₇H₁₇BrN₂O: C 72.58%, H 8.34%, N 8.91%. Found: C 72.75%, H 8.66%, N 8.50%.

### Example 18: (3-chloro-4-methoxyphenyl)((4R,4aR,5aS,6S,6aS)-3,3a,4,4a,5,5a,6,6a-octahydro-4,6-ethenocyclopropa[f]isoindol-2(1H)-yl)methanone

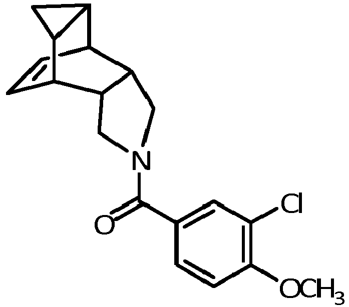

To a solution of 4-azapentacyclo[5.3.2.0^{2,6}.0^{8,10}]dodec-11-ene hydrochloride (200 mg, 1.03 mmol) in EtOAc (10 mL) were added 3-chloro-4-methoxybenzoic acid (176 mg, 0.94 mmol), HOBt (190 mg, 1.41 mmol), EDC (218 mg, 1.41 mmol) and triethylamine (0.6 mL, 4.14 mmol). The reaction mixture was stirred at room temperature for 24 h. To the resulting suspension was then added water (10 mL) and the phases were separated. The organic phase was washed with saturated aqueous NaHCO₃ solution (10 mL) and brine (10 mL), dried over anh. Na₂SO₄ and filtered. Evaporation in *vacuo* of the organics gave the title compound as a yellow semisolid (329 mg, quantitive yield). The analytical sample was obtained by column chromatography (Hexane/Ethyl acetate) to obtain the title compound as a waxy solid (164 mg). IR (ATR) v: 615, 651, 692, 708, 754, 814, 835, 850, 891, 915, 946, 1018, 1059, 1095, 1142, 1183, 1232, 1256, 1294, 1349, 1387, 1418, 1501, 1560, 1599, 1617, 2868, 2925, 3002 cm⁻¹. Accurate mass: Calculated for [C₁₉H₂₀ClNO₂+H]⁺: 300.1255. Found: 300.1251.

### Example 19: (6-bromopyridin-2-yl)((4R,4aR,5aS,6S,6aS)-3,3a,4,4a,5,5a,6,6a-octahydro-4,6-ethenocyclopropa[f]isoindol-2(1H)-yl)methanone

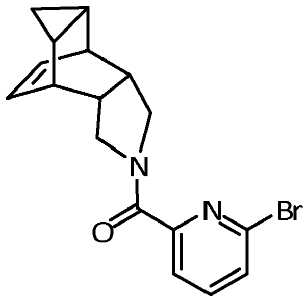

To a solution of 4-azapentacyclo[5.3.2.0^{2,6}.0^{8,10}]dodec-11-ene hydrochloride (300 mg, 1.55 mmol) in EtOAc (15 mL) were added 6-bromopyridine-2-carboxylic acid (285 mg, 1.41 mmol), HOBt (286 mg, 1.41 mmol), EDC (328 mg, 1.41 mmol) and triethylamine (0.9 mL, 6.20 mmol). The round bottomed flask was wrapped with aluminium foil and the reaction mixture was stirred at room temperature for 24 h. To the resulting suspension was then added water (15 mL) and the phases were separated. The organic phase was washed with saturated aqueous NaHCO₃ solution (15 mL) and brine (15 mL), dried over anh. Na₂SO₄ and filtered. Evaporation in *vacuo* of the organics gave the title compound as a yellowish solid (323 mg, 66.3 % yield). The analytical sample was obtained by crystallization from hot EtOAc (209 mg), m. p. 185-186 °C. IR (ATR) v: 643, 659, 705, 734, 762, 814, 827, 850, 912, 935, 984, 1044, 1080, 1119, 1165, 1199, 1225, 1245, 1274, 1305, 1341, 1392, 1405, 1454, 1545, 1576, 1617, 2857, 2925, 3002, 3043, 3059 cm⁻¹. Elemental analysis: Calculated for C₁₇H₁₇BrN₂O: C 59.14%, H 4.96%, N 8.11%. Found: C 59.31 %, H 4.92%, N 7.87%.

### Example 20: (6-(4-hydroxyphenyl)pyridin-2-yl)((4R,4aR,5aS,6S,6aS)-3,3a,4,4a,5,5a,6,6a-octahydro-4,6-ethenocyclopropa[f]isoindol-2(1H)-yl)methanone

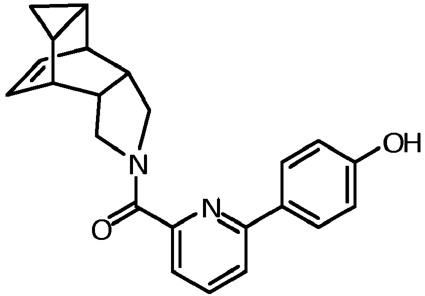

A mixture of the compound obtained in Example 19 (300 mg, 0.87 mmol), 4-hydroxyphenylboronic acid (132 mg, 0.96 mmol), Pd(Ph₃)₄ (10 mg, 0.009 mmol) and K₂CO₃ (240 mg, 1.74 mmol) in dioxane (3 mL) and water (1.5 mL) was heated at 100° C for 2 h. Then EtOAc (10 mL) was added and the organic phase was washed with water (10 mL), dried over anh. Na₂SO₄ and filtered. Evaporation in *vacuo* of the organics gave a mixture of the product and starting materials (461 mg). Column chromatography (Hexane/Ethyl acetate) gave the title compound as a white solid (184 mg, 59.0 % yield), m. p. 221-222 °C. IR (ATR) v: 630, 654, 711, 754, 822, 840, 915, 946, 992, 1039, 1085, 1103, 1173, 1230, 1276, 1297, 1346, 1380, 1400, 1429, 1460, 1516, 1558, 1586, 1604, 2930, 3002, 3126 cm⁻¹. Accurate mass: Calculated for [C₂₃H₂₂N₂O₂+H]⁺: 359.1754. Found: 359.1750.

### Example 21: (6-((2-hydroxyethyl)amino)pyridin-3-yl)((4R,4aR,5aS,6S,6aS)-3,3a,4,4a,5,5a,6,6a-octahydro-4,6-ethenocyclopropa[f]isoindol-2(1H)-yl)methanone

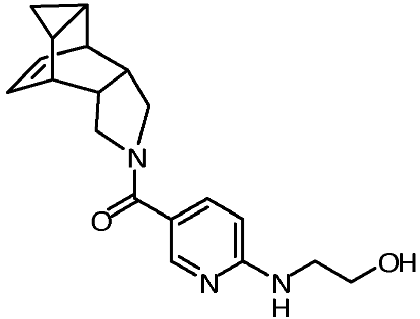

A solution of the compound obtained in example 11 (100 mg, 0.33 mmol) and ethanolamine (0.36 mL, 6 mmol) was stirred at 120 °C for 24 hours. Water (10 mL) and EtOAc (10 mL) were added and the phases were separated. The organic phase was dried over anh. Na₂SO₄, filtered and evaporated in *vacuo* to give the title compound as a yellowish solid (65 mg, 60.7% yield). The analytical sample was obtained by crystallization from hot EtOAc (59 mg), m. p. 87-88 °C. IR (ATR) v: 636, 716, 731, 767, 811, 832, 909, 943, 984, 1010, 1044, 1070, 1150, 1232, 1274, 1302, 1343, 1423, 1460, 1488, 1527, 1591, 1612, 2868, 2914, 3002, 3064, 3136, 3271 cm⁻¹. Accurate mass: Calculated for [C₁₉H₂₃N₃O₂+H]⁺: 326.1863. Found: 326.1867.

### Example 22: 1-(5-((4R,4aR,5aS,6S,6aS)-1,2,3,3a,4,4a,5,5a,6,6a-decahydro-4,6-ethenocyclopropa[f]isoindole-2-carbonyl)pyridin-2-yl)piperidine-4-carboxamide

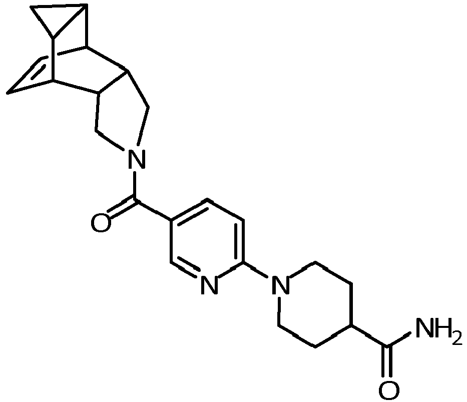

To a solution of the compound obtained in example 11 (100 mg, 0.33 mmol) and isonipecotamide (85 mg, 0.66 mmol) in DMF (0.5 mL) was added solid K₂CO₃ (82 mg, 0.59 mmol). The resulting suspension was stirred at 90 °C for 48 hours. Water (5 mL) and DCM (5 mL) were added and the phases were separated. The aqueous phase was then extracted with further DCM (2 x 5 mL). The organics were dried over anh. Na₂SO₄, filtered and evaporated in *vacuo* to give the title compound as a yellowish solid (110 mg, 84.6% yield), m. p. 162-163 °C. IR (ATR) v: 630, 692, 723, 773, 811, 845, 943, 982, 1010, 1028, 1041, 1095, 1129, 1178, 1219, 1238, 1312, 1351, 1367, 1408, 1431, 1501, 1540, 1584, 1599, 1687, 1736, 2919, 3152, 3307 cm⁻¹. Accurate mass: Calculated for [C₂₃H₂₈N₄O₂+H]⁺: 393.2285. Found: 393.2285.

### Example 23: ((4R,4aR,5aS,6S,6aS)-3,3a,4,4a,5,5a,6,6a-octahydro-4,6-ethenocyclopropa[f]isoindol-2(1H)-yl)(6-(thiophen-3-yl)pyridin-2-yl)methanone

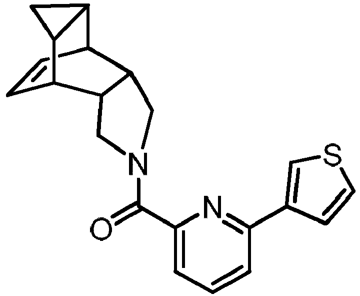

A mixture of the compound obtained in Example 19 (230 mg, 0.67 mmol), 3-thiopheneboronic acid (93 mg, 0.73 mmol), Pd(Ph₃)₄ (8 mg, 0.007 mmol) and K₂CO₃ (185 mg, 1.34 mmol) in dioxane (2.3 mL) and water (1.3 mL) was heated at 100° C for 2 h. Then EtOAc (10 mL) was added and the organic phase was washed with water (10 mL), dried over anh. Na₂SO₄ and filtered. Evaporation in *vacuo* of the organics gave a mixture of the product and starting materials (238 mg). Column chromatography (Hexane/Ethyl acetate) gave the title compound as a yellowish foamy solid (184 mg, 79.0 % yield), m. p. 47-48 °C. IR (ATR) v: 615, 646, 703, 716, 752, 791, 829, 845, 863, 915, 948, 987, 1036, 1095, 1178, 1235, 1274, 1343, 1418, 1431, 1460, 1566, 1581, 1617, 1971, 2000, 2051, 2175, 2325, 2868, 2919, 2997 cm⁻¹. Accurate mass: Calculated for [C₂₁H₂₀N₂OS+H]⁺: 349.1369. Found: 349.1374.

### Example 24: ((4R,4aR,5aS,6S,6aS)-octahydro-4,6-ethanocyclopropa[f]isoindol-2(1H)-yl)(phenyl)methanone

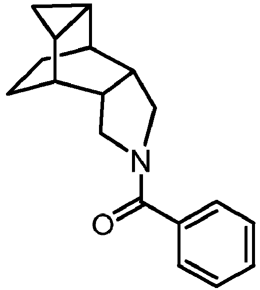

A solution of the compound from example 5 (80 mg) and Pd/C (16 mg) in absolute EtOH (12 mL) was hydrogenated at 1 atm for 24 hours. The suspension was filtered and concentrated in vacuo to give the title compound as a white solid (79 mg, 98.0% yield), m. p. 79-80 °C. IR (ATR) v: 644, 685, 711, 729, 785, 845, 871, 935, 951, 1010, 1026, 1046, 1075, 1147, 1160, 1248, 1292, 1341, 1418, 1444, 1480, 1576, 1617, 1888, 1961, 2005, 2129, 2201, 2351, 2857, 2925, 2992 cm⁻¹. Accurate mass: Calculated for [C₁₈H₂₁NO+H]⁺: 268.1696. Found: 268.1703.

### Example 25: Pharmacological data of selected compounds

The inhibitory activity of the compounds of the invention for the enzymes 11β-HSD1 and 11β-HSD2 may be tested with the following protocols:

### Determination of the 11β-HSD1 inhibition in Human Liver Microsomes

11β-HSD1 activity was determined in mixed sex, Human Liver Microsomes (Celsis In-vitro Technologies) by measuring the conversion of ³H-cortisone to ³H-cortisol. Percentage inhibition was determined relative to a no inhibitor control. 5 µg of Human Liver microsomes was pre-incubated at 37°C for 10 minutes with inhibitor (assay concentrations of 1, 0.5, 0.1, 0.05, 0.01, 0.005, 0.001 and 0.0005 µM) and 1 mM NADPH in a final volume of 90 µL Krebs buffer. 10 µL of 200 nM ³H-cortisone was then added followed by incubation for at 37°C for a further 30 minutes. The assay was terminated by rapid freezing on dry ice. ³H-cortisone to ³H-cortisol conversion was determined in 50 µL of the defrosted reaction by capturing liberated ³H-cortisol on 100 µL 1:8000 dilution anti-cortisol (HyTest Ltd)-coated scintillation proximity assay beads (protein A-coated YSi, GE Healthcare). The percentages of inhibition at every tested concentration were analyzed using a non-linear regression curve fitting (variable slope) by using the software GraphPad Prism 5.0.

### Determination of 11β-HSD1 inhibition in HEK293 cells stably transfected with the 11β-HSD1 gene

11β-HSD1 activity was determined in cells that are incubated with substrate (cortisone) and product (cortisol) is determined by LC/MS. Cells are plated at 2 x 104 cells/well in a 96-well tissue culture microplate and incubated overnight at @370 C in 5% CO₂ 95% O₂. Compounds to be tested are solubilized in 100% DMSO at 10 mM and serially diluted in water and 10% DMSO to final concentration 100 µM in 10% DMSO. 10 µl of each test dilution is dispensed into the well of a 96-well microplate (assay concentration of 10 µM). Low and high control wells contain 10 µl of 10% DMSO. A solution of DMEM is prepared containing 1% penicillin, 1% streptomycin and 300 nM cortisone. Medium is removed from cell assay plate and DMEM solution (100 µl) added to each well. Cells are incubated for 2 hours @370 C in 5% CO₂ 95% O₂. Following incubation medium is removed from each well into 500 µl ethyl acetate mixed by vortex and incubated at room temperature for 5 minutes. A calibration curve of known concentrations of cortisol in assay medium is also set up and added to 500 µl ethyl acetate vortexed and incubated as above. The supernatant is removed to a 96 deep well plate and dried down under liquid nitrogen at 65°C. Each well is solubilised in 100 µl 70:30 H2O:ACN and removed to a 96 well v bottomed plate for LC/MS analysis.

### Determination of 11β-HSD2 inhibition in HEK293 cells stably transfected with the 11β-HSD2 gene

11β-HSD2 activity was determined in cells that are incubated with substrate (cortisol) and product (cortisone) is determined by LC/MS. Cells are plated at 2 x 10⁴ cells/well in a 96-well tissue culture microplate and incubated overnight at @37° C in 5% CO₂ 95% O₂. Compounds to be tested are solubilized in 100% DMSO at 10 mM and serially diluted in water and 10% DMSO to final concentration in 10% DMSO. 10 µl of each test dilution is dispensed into the well of a 96-well microplate (assay concentrations of 10, 1, 0.1 µM). Low and high control wells contain 10 µl of 10% DMSO. A solution of DMEM is prepared containing 1% penicillin, 1% streptomycin and 300 nM cortisone. Medium is removed from cell assay plate and DMEM solution (100 µl) added to each well. Cells are incubated for 2 hours @37° C in 5% CO₂ 95% O₂. Following incubation medium is removed from each well into 500 µl ethyl acetate mixed by vortex and incubated at room temperature for 5 minutes. A calibration curve of known concentrations of cortisone in assay medium is also set up and added to 500 µl ethyl acetate vortexed and incubated as above. The supernatant is removed to a 96 deep well plate and dried down under liquid nitrogen at 65°C. Each well is solubilised in 100 µl 70:30 H₂O:AC and removed to a 96 well v bottomed plate for LC/MS analysis.

Following the above described protocols the inhibitory activity of compounds of the present invention has been reported and is shown in the following table 1:

**TABLE 1**

| Example | HSD1 | | HSD2 |
|---|---|---|---|
| | IC₅₀ in HLM^{a} | % inh in cells (10 µM) | IC₅₀ in cells^{b} |
| 1 | A | 100 | C |
| 2 | A | 100 | A |
| 7 | C | 85 | C |
| 9 | A | 77 | B |
| 10 | A | 41 | B |
| 15 | B | 100 | C |

| | | | |
|---|---|---|---|
| ^{a} In the column "HSD1 IC₅₀ in HLM" A means that IC₅₀ is lower than 50 nM, B means that IC₅₀ is at least 50 nM but less than 500 nM and C means that IC₅₀ is at least 500 nM but less than < 1000 nM. ^{b} In the column "HSD2 IC₅₀ in cells" A means that IC₅₀ is lower than 100 nM, B means that IC₅₀ is at least 100 nM but less than 1000 nM and C means that IC₅₀ is at least 1000 nM. | | | |

## Claims

1. A compound of formula (I): 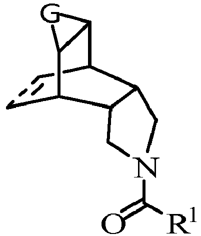 wherein
R¹ is selected from the group consisting of:
a) C₃₋₈ cycloalkyl, optionally substituted with 1 to 3 groups selected from halogen atoms and C₁₋₄ alkyl;
b) C₃₋₈ cycloalkenyl, optionally substituted with 1 to 3 groups selected from halogen atoms and C₁₋₄ alkyl;
c) C₆₋₁₀ aromatic group optionally substituted with 1 to 3 groups selected from halogen atoms, CN, CF₃, NH₂, OH, C₁₋₄ alkyl and C₁₋₄ alkoxy;
d) C₅₋₁₀ heteroaromatic group optionally substituted with 1 to 3 groups selected from halogen atoms, CN, CF₃, NHR³, C₁₋₄ alkyl, C₁₋₄ alkoxy, C₅₋₁₀ heteroaryl (optionally substituted with 1 to 3 groups selected from halogen atoms, CN, CF₃, NH₂, OH, C₁₋₄ alkyl and C₁₋₄ alkoxy), phenyl (optionally substituted with one or two groups selected from halogen atoms, CN, OH, CF₃, C₁₋₄ alkyl and C₁₋₄ alkoxy) and C₅₋₇ heterocyclyl (optionally substituted with a group selected from -COR⁴, -COOR⁴, -CN, -CONHR⁴,-CONH₂ and phenyl which is optionally substituted with one or two groups selected from halogen atoms, CONH₂, CN, OH, CF₃, C₁₋₄ alkyl and C₁₋₄ alkoxy), wherein the C₅₋₇ heterocyclyl group comprises a nitrogen atom forming part of the heterocycle and is attached to the C₅₋₁₀ heteroaromatic group through said nitrogen atom;
e) C₃₋₈ heterocyclyl optionally substituted with a group selected from C₁₋₄ alkyl, C₃₋₈ cycloalkyl and -COR⁵; and
f) -C(CH₃)₂-R²;
G is a rest selected from the group consisting of -CH₂-, -CH₂-CH₂- and -CH=CH-;
R² is selected from the group consisting of hydrogen atom and C₁₋₃ alkyl
R³ is selected from the group consisting of C₁₋₃ alkyl and C₁₋₃ hydroxyalkyl
R⁴ is selected from the group consisting of C₁₋₆ alkyl and phenyl
R⁵ is a group C₁₋₆ alkyl
the bond represented by -̅-̅-̅-̅ is either a single bond or a double bond;
and pharmaceutically stable salts thereof.

2. A compound according to claim 1, wherein the rest 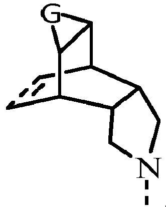 is selected from the group consisting of rests (A) to (D):

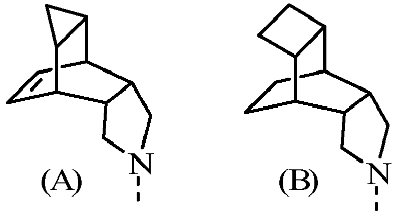

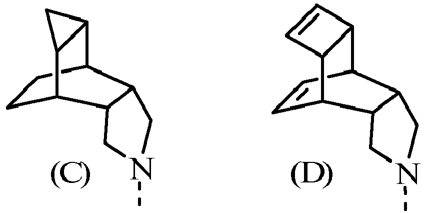

3. A compound according to claim 2, wherein the rest 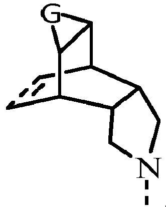 is selected from the group consisting of rests (A) and (C):

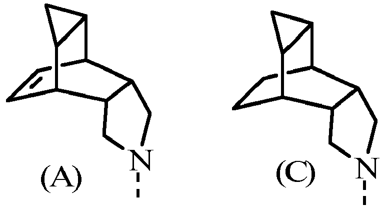

4. A compound according to anyone of claims 1 to 3, wherein G is a rest -CH₂-.

5. A compound according to anyone of claims 1 to 4, wherein the bond represented by -̅-̅-̅-̅ is a double bond.

6. A compound according to anyone of claims 1 to 4, wherein R¹ is a C₃₋₈ cycloalkyl, optionally substituted with 1 to 3 groups selected from halogen atoms and C₁₋₄ alkyl.

7. A compound according to anyone of claims 1 to 4, wherein R¹ is a C₃₋₈ cycloalkenyl, optionally substituted with 1 to 3 groups selected from halogen atoms and C₁₋₄ alkyl.

8. A compound according to anyone of claims 1 to 4, wherein R¹ is a C₆₋₁₀ aromatic group optionally substituted with 1 to 3 groups selected from halogen atoms, CN, CF₃, NH₂, OH, C₁₋₄ alkyl and C₁₋₄ alkoxy.

9. A compound according to anyone of claims 1 to 4, wherein R¹ is a C₅₋₁₀ heteroaromatic group optionally substituted with 1 to 3 groups selected from halogen atoms, CN, CF₃, NHR³, C₁₋₄ alkyl, C₁₋₄ alkoxy, C₅₋₁₀ heteroaryl (optionally substituted with 1 to 3 groups selected from halogen atoms, CN, CF₃, NH₂, OH, C₁₋₄ alkyl and C₁₋₄ alkoxy), phenyl (optionally substituted with one or two groups selected from halogen atoms, CN, OH, CF₃, C₁₋₄ alkyl and C₁₋₄ alkoxy) and C₅₋₇ heterocyclyl (optionally substituted with a group selected from -COR⁴, -COOR⁴, -CN, -CONHR⁴, -CONH₂ and phenyl which is optionally substituted with one or two groups selected from halogen atoms, CONH₂, CN, OH, CF₃, C₁₋₄ alkyl and C₁₋₄ alkoxy), wherein the C₅₋₇ heterocyclyl group comprises a nitrogen atom forming part of the cycle and is attached to the C₅₋₁₀ heteroaromatic group through said nitrogen atom.

10. A compound according to anyone of claims 1 to 4, wherein R¹ is a C₃₋₈ heterocyclyl optionally substituted with a group selected from C₁₋₄ alkyl, C₃₋₈ cycloalkyl and COR⁵ wherein R⁵ is a group C₁₋₆ alkyl.

11. A compound according to anyone of claims 1 to 4, wherein R¹ is a group -C(CH₃)₂-R², wherein R² is selected from the group consisting of hydrogen atom and C₁₋₃ alkyl.

12. A compound according to anyone of claims 1 to 11 for use as a medicament.

13. A compound according to anyone of claims 1 to 11 for use in the treatment or prevention of a disease or conditions susceptible of improvement by inhibition of 11β-hydroxysteroid dehydrogenase type 1 (11β-HD1).

14. A compound for use according to claim 13 wherein the disease or condition susceptible of improvement by inhibition of 11β-hydroxysteroid dehydrogenase type 1 (11(3-HD1) is selected from the group consisting of obesity, insulin resistance, metabolic syndrome, cognitive disfunction associated to Alzheimer's disease, HIV-associated dementia, Creutzfeld-Jakob disease, Parkinson's disease, Huntington's disease, vascular inflammation, atherosclerosis, diabetes type 2, cardiovascular disease, glaucoma, osteoporosis, myocardial infarction and alcohol abuse treatment.
